# EUROPEAN PATENT SPECIFICATION

(11) **EP 0 501 797 B1**
(45) Date of publication and mention of the grant of the patent: **05.06.1996**
(21) Application number: 92301654.7
(22) Date of filing: 27.02.1992
(51) Int. Cl.: C07D 311/68, C07D 311/70, A61K 31/35

(54) **Pyranyl cyanoguanidine derivatives**
Pyranylcyanoguanidinderivate
Dérivés de pyranylcyanoguanidine

(30) Priority: 27.02.1991 US 661763
(43) Date of publication of application: 02.09.1992
(73) Proprietor: E.R. SQUIBB & SONS, INC., PrincetonNew Jersey 08543-4000 (US)
(72) Inventor: Atwal, Karnail, Newtown, PA (US); Kim, Kyoung S., Lawrenceville, NJ (US); Grover, Gary J., Stockton, NJ (US)
(74) Representative: Thomas, Roger Tamlyn

(56) References cited:
- EP-A- 0 344 747
- EP-A- 0 359 537
- EP-A- 0 389 861
- EP-A- 0 401 010
- EP-A- 0 412 531

## Description

The present invention relates to novel compounds having potassium channel activating activity which are therefore useful, for example, as cardiovascular agents. More specific uses are hereinafter referred to in the claims.

In accordance with the present invention novel compounds having potassium channel activating activity which are useful, for example, as cardiovascular agents, are disclosed. These compounds have the general formula 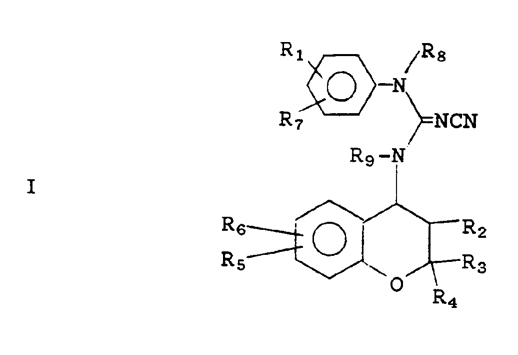
R₁ and R₇ are each independently hydrogen, alkyl, cyano, alkoxy, nitro, halo, hydroxy, haloalkyl, benzyloxy, with the proviso that at least one of R₁ and R₇ is other than hydrogen;
R₂ is hydrogen, hydroxy, 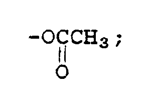
R₃ and R₄ are each independently hydrogen, alkyl or arylalkyl, or, R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO₂, -COR, - COOR, -CONHR, -CONR_{z}, -CF₃, S-alkyl, -SOalkyl, -SO₂alkyl, 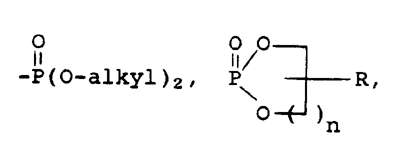 halogen, amino, substituted amino, O-alkyl, OCF₃, OCH₂CF₃, -OCOalkyl, -OCONRalkyl, -NRCOalkyl and NRCOOalkyl, NRCONR₂ wherein R in each of the above groups can be hydrogen, alkyl, haloalkyl, aryl, arylalkyl, cycloalkyl, or (cycloalkyl)alkyl;
R₆ is selected from H, alkyl, OH, O-alkyl, amino, substituted amino, NHCOR (wherein R is as defined above), CN, and N0₂;
R₈ is selected from hydrogen, alkyl;
Rg is selected from hydrogen, alkyl, alkenyl, aryl, arylalkyl, cycloalkyl or cycloalkylalkyl; and
n is 1, 2 or 3.

### Detailed Description of the Present Invention

This invention relates to the cyanoguanidine compounds of formula I above, to compositions and the methods of using such compounds. The compounds of formula I are useful, for example, as cardiovascular agents. Preferred compounds are those with the 3S, 4R stereochemistry

EP-A-412531 discloses potassium channel activators of generally similar formula except that NR⁸ is a single bond or an alkylene group and the phenyl group is unsubstituted.

EP -A-401010 generally discloses compounds having an unsubstituted phenyl group attached to the N-atom, that is, compounds similar to the present formula I but where R₁ and R₇ are each hydrogen. It has now been found that substitution on this phenyl ring provides selective antiischemic agents which surprisingly have a longer duration of action than their unsubstituted counterparts. It has been found that the present compounds are useful for preventing or alleviating ischemic damage which results from organ surgery procedures, e.g., bypass and transplant.

The term "alkyl" used in defining various symbols refers to straight or branched chain saturated hydrocarbon radicals having up to eight carbons, preferably from one to five carbons. Similarly, the terms "alkoxy" and "alkylthio" refer to such alkyl groups attached to an oxygen or sulfur.

The term "alkenyl" refers to straight or branched chain hydrocarbon radicals having from two to eight carbons and one double bond, preferably three to five carbons. The term "alkynyl" refers to straight or branched chain hydrocarbon radicals having from two to eight carbons and one triple bond, preferably three to five carbons.

The term "cycloalkyl" refers to saturated carbocyclic rings of 3 to 7 carbon atoms with cyclopropyl, cyclopentyl and cyclohexyl being most preferred.

The term "halo" or "halogen" refers to chloro, bromo and fluoro.

The term "halo substituted alkyl" refers to such alkyl groups described above in which one or more hydrogens have been replaced by chloro, bromo or fluoro groups such as trifluoromethyl, which is preferred,pentafluoroethyl, 2,2,2-trichloroethyl, chloromethyl, bromomethyl, etc.

The term "aryl" refers to phenyl, 1-naphthyl, 2-naphthyl; mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons, -CF₃, -OCHF₂, 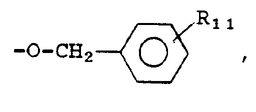 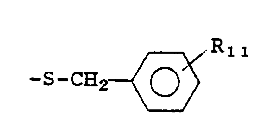
(wherein R₁₁ is hydrogen,
alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl; and di-substituted phenyl, 1-naphthyl, 2-naphthyl wherein said substituents are selected from methyl, methoxy, methylthio, halo, CF₃, nitro, amino, and OCHF₂.

Preferred aryl groups include unsubstituted phenyl and monosubstituted phenyl wherein the substituents are nitro, halo, -CF₃, alkyl, cyano or methoxy.

The term "substituted amino" refers to a group of the formula -NZₗZ₂ wherein Z₁ is hydrogen, alkyl, cycloalkyl, aryl, arylalkyl or cycloalkylalkyl, and Z₂ is alkyl, cycloalkyl, aryl, arylalkyl or cycloalkylalkyl, or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy The compounds of formula I can be prepared by treatment of a thiourea of the formula 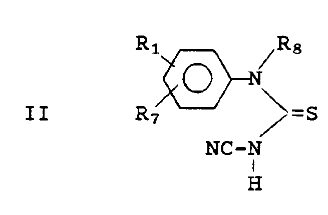 with an amine of the formula 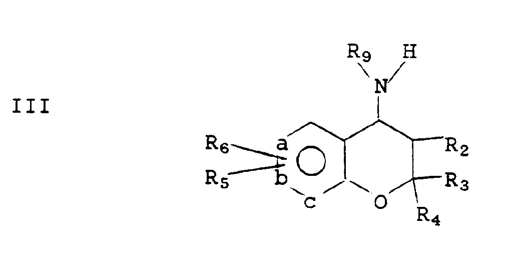 in the presence of a coupling agent, such as a carbodiimide, in a solvent, such as dimethylformamide, tetrahydrofuran, acetonitrile or dichloromethane. If dicyclohexylcarbodiimide is used, it should be employed with an acid source. Preferably, the carbodiimide is of the formula 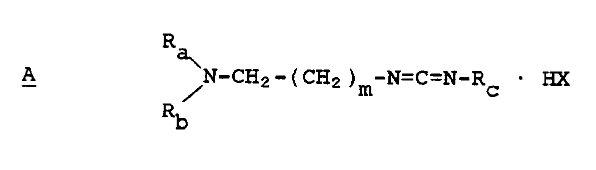 wherein X is halogen, Rₐ, R_{b} and R_{c} are independently alkyl, cycloalkyl, phenyl, phenylalkyl, cycloalkylalkyl or Rₐ and R_{b} together with the N-atom form 1-pyrrolidinyl, 1-piperidinyl, 4-morpholinyl, 4-thiomorpholinyl, 4-alkyl-1-piperazinyl or 4-phenylalkyl-1-piperazinyl. Most preferably the carbodiimide is 1-(3-dimethylaminopropyl)-3-ethylcarbodiimide hydrochloride.

The thiourea of formula II, wherein R₈ is hydrogen can be prepared by heating an isothiocyanate of the formula 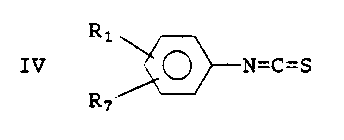 with either monosodium cyanamide or with cyanamide in the presence of an organic base, such as triethyl amine.

The other thioureas of formula II can be prepared by standard methods described in the literature, such as by C. R. Rasmussen, F. J. Villani, Jr., L. E. Weaner, B. E. Reynolds, A. R. Hood, L. R. Hecker, S. O. Nortey, A. Hanslin, M. J. Costanzo, E. T Powell, A. J. Molinari, Synthesis, 1988, p. 456, and V. V. Mozolis and S. P. Locubaitite, Russian Chemical Reviews. 1973, 42, 587.

The aminoalcohol of formula III wherein R₂ is hydroxy can be prepared by methods described in the literature, such as by J. M. Evans, C. S. Fake, T C. Hamilton, R. H. Poyser, E. A. Watts, J. Med. Chem. 1983, 26, 1582 and J. Med. Chem. 1986,29,2194; R. W. Lang, P. F. Wenk, Helvetica Chimica Acta, 1988, 71, 596; EP 0205292 A2 (1986), and WO 87/07607.

The amine of formula III, wherein R₂ is hydrogen, can be prepared from a ketone of the formula 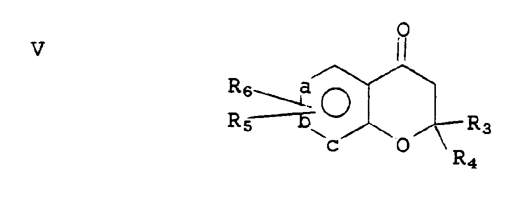 by standard methodology The ketone of formula V can be obtained by literature procedures, such as disclosed by P. Sebok and T Timar, Heterocycles, 1988, 27, 2595; P. Teixidor et al., Heterocycles, 1988, 27, 2459; A. Benerji and N. C. Goomer, Tetrahedron Letters, 1979, 3685; G. Ariamala and K. K. Subramanian, Tetrahedron Letters. Vol. 29, No. 28, p. 3487-3488 (1988).

The compounds of formula I can also be prepared by heating a thiourea of the formula 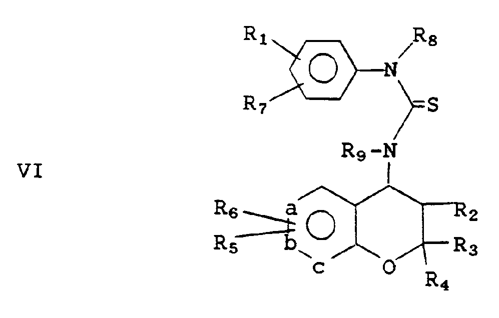 with monosodium cyanamide in the presence of a carbodiimide such as 1-(3-dimethylaminopropyl)-3-ethylcarbodiimide or dicyclohexylcarbodiimide in an organic solvent.

The compounds of formula VI can be prepared from the amino alcohol of formula III by standard methods (i.e., the Rasmussen and Mozolis references above).

The compounds of formula I can also be prepared by reacting a compound of the formula 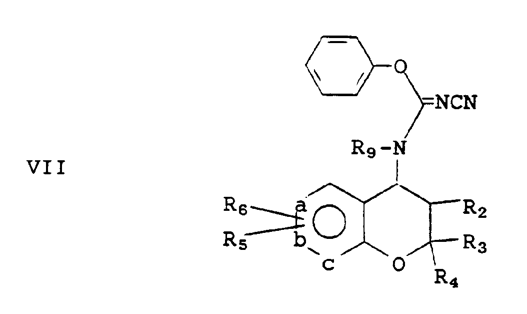 with an amine of the formula 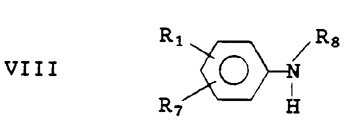 in a polar solvent such as isopropanol or in the presence of trimethyl aluminum in a polar aprotic solvent, such as dichloromethane. The compounds of formula VII are prepared by reacting an amine of formula III with diphenylcyano- carbonimidate.

The compounds of the present invention wherein R₂ is OCOalkyl can be prepared by acylation of the alcohol of formula I, wherein R₂ is OH, with an acid chloride of the formula 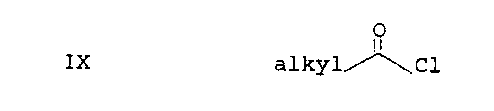 in the presence of a base catalyst, such as pyridine or triethylamine.

For the preparation of individual enantiomers of compounds of formula I (wherein R₂ =H, OH), compound II (R2 = =H, OH) is converted to diastereomeric amides of the formula 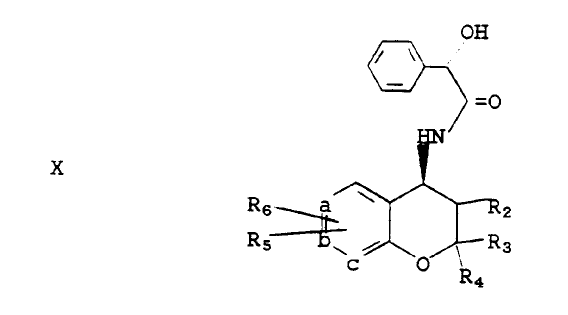 and 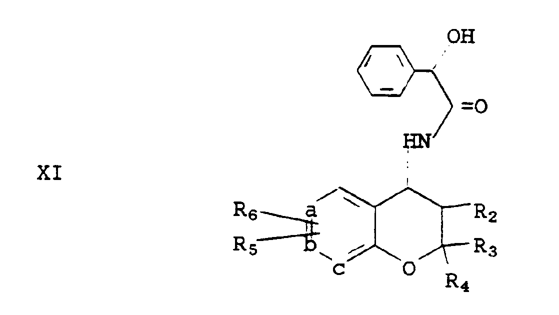 by treatment with chiral nonracemic mandelic acid in the presence of dicyclohexylcarbodiimide.

Compounds X and XI are separated by crystallization or chromatography The enantiomer of mandelic acid that yields crystalline diastereomer with the desired 4R-stereochemistry of benzopyran (as shown in formula X) is preferred in the resolution step.

Compounds X and XI are then hydrolyzed by heating in the presence of sulfuric acid in dioxane to give enantiomers of the formula 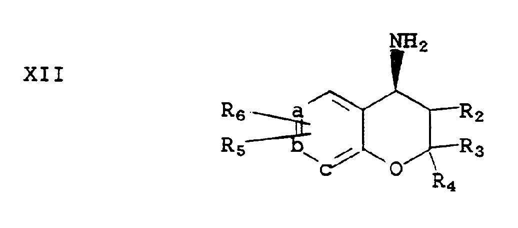 and 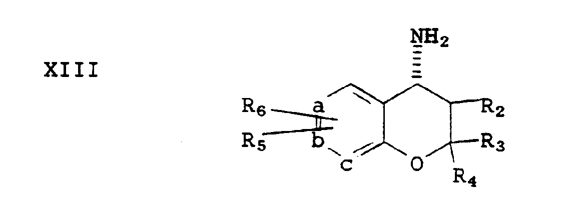 The enantiomers XII and XIII are then converted to chiral nonracemic compounds of formula I using the methodologies described previously

The compounds of the present invention can have asymmetric centers at carbons 2-4 of benzopyran ring. Also, any one of the R's can have an asymmetric carbon. Consequently, compounds of formula I can exist in diastereomeric forms or in mixtures thereof. The above described process can utilize racemates, enantiomers or diastereomers as starting materials. When diastereomeric products are prepared, they can be separated by conventional chromatographic or fractional crystallization methods.

The compounds of the present invention wherein Rg and/or R₈ is hydrogen, can exist as a mixture of tautomers represented by the following structures. The tautomeric products are obtained in relative amounts that differ from compound to compound. All forms are included in the scope of formula I.

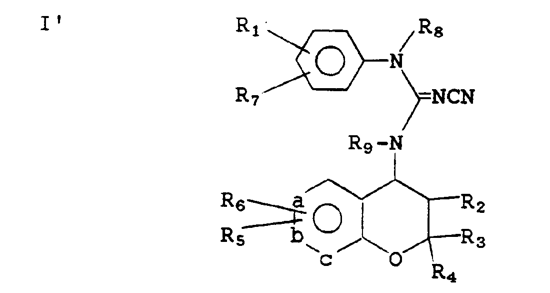

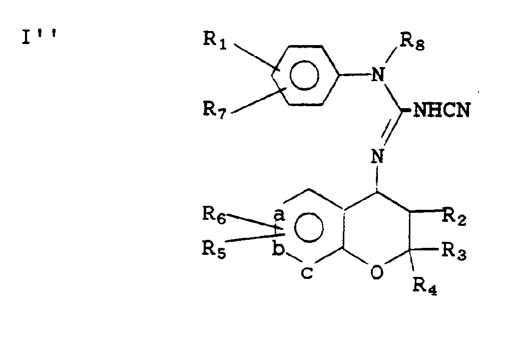

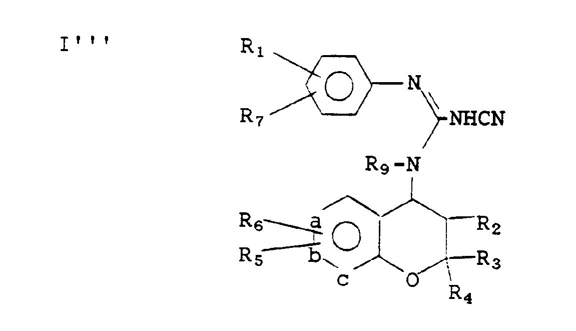

The compounds of the present invention are "ischemia selective" in that they have little or no vasodilatory activity in normal tissue but are potassium channel activators in ischemic tissue. Thus, they are useful for the treatment of ischemic conditions such as myocardial ischemia, cerebral ischemia, lower limb ischemia and the like. This selectivity of action means that in the treatment of ischemic heart, these compounds are less likely to cause coronary steal, profound hypotension and coronary underperfusion. By "little or no vasodilator activity" is meant that these compounds have IC₅₀ (rat aorta) values greater than that of the known potassium channel activator, cromakalim. Preferred compounds for ischemia have IC₅₀ (methoxamine contracted rat aorta) values greater than that of cromakalim, especially >10 times that of cromakalim (i.e., have 1/10 the vasodilatory action of cromakalim) and most preferred are those compounds having IC₅₀ (rat aorta) values >50 times that of cromakalim.

Thus, for example, by the administration of a composition containing one (or a combination) of the compounds of this invention, ischemic conditions of a mammalian (e.g., human) host are reduced. A single dose, or preferably two to four divided daily doses, provided on a basis of about 0.001 to 100 mg per kilogram of body weight per day, preferably from about 0.1 to about 25 mg per kilogram per day, is appropriate to reduce ischemic conditions. The substance is preferably administered orally, but parenteral routes, such as the subcutaneous, intramuscular, or intravenous routes or any other convenient delivery system, such as inhalation or intranasal solutions or transdermal patches, can also be employed. The above doses are also suitable for the other cardiovascular (e.g., hypertension) and non-cardiovascular uses.

The compounds of this invention can also be formulated in combination with a diuretic such as, chlorothiazide, hydrochlorothiazide, flumethiazide, hydroflumethiazide, bendroflumethiazide, methylchlothiazide, trichloromethiazide, polythiazide or benzthiazide as well as ethacrynic acid, tricrynafen, chlorthalidone, furosemide, musolimine, bumetanide, triamterene, amiloride and spironolactone and salts of such compounds, angiotensin converting enzyme inhibitors such as captopril, zofenopril, fosinopril, enalapril, ceranopril, cilazopril, delapril, pentopril, quinapril, ramipril, lisinopril, and salts of such compounds, thrombolytic agents such as tissue plasminogen activator (tPA), recombinant tPA, streptokinase, urokinase, prourokinase, and anisoylated plasminogen streptokinase activator complex (APSAC, Eminase, Bee- cham Laboratories), or calcium channel blocking agents such as nifedipine or diltiazem. Such combination products if formulated as a fixed dose employ the compounds of this invention within the dose range described above and the other pharmaceutically active agent within its approved dose range.

The compounds of formula I, and combinations thereof, can be formulated, as described above, in compositions such as tablets, capsules or elixirs for oral administration, in sterile solutions or suspensions for parenteral administration, and may also be administered via transdermal patch or nasal inhalation solutions. About 10 to 500 milligrams of a compound of formula I is compounded with physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

As mentioned above the compounds herein have also been found useful in preventing/alleviating tissue and cell damage in organ surgery procedures, e.g., bypass and transplant.

Cardiopulmonary bypass and heart transplant are two important surgical procedures used by cardiac surgeons. While they both are designed to improve cardiac functional status, the techniques could be greatly improved. In both cases, the procedures require that the hearts be removed from the normal circulation of the body and thus by definition, some degree of damage may be observed. In bypass and transplant, cardioplegic solution, rather than blood, are employed to perfuse the coronary arteries. Accordingly, the conditions and attendant risks/damage resulting from these procedures may differ from coronary stenosis induced damage. To reduce the degree of surgical damage, the hearts are perfused in a retrograde fashion with a cardioplegic solution designed to reduce energy needs of the tissue by arresting the hearts, making them hypothermic (reduce energy demands) and also supplying them with essential substrates.

In carrying out cardiopulmonary bypass and heart transplant according to the present invention, a potassium channel activator is added to any solution used to perfuse the coronary arteries or used in connection with bypass and transplant procedures. These solutions may be selected from any of the various cardioplegic solutions, intracellular solutions, etc., which are used to perfuse the arteries, to store the organ, to arrest the heart for transplant, etc. Additionally, the present invention encompasses administration of a potassium channel activator to a mammalian specie, i.e., monkey, dog, cat, rat, human, etc., which is involved in the bypass or transplant procedure. For example, a potassium channel activator can be administered to a bypass patient, organ donor and/or organ recipient before, during and/or after the bypass or transplant procedure.

While the present invention relating to transplant procedures is most frequently described in terms of heart transplant, the methods of this invention are meant to include other types of organ transplant as well. Organ transplant procedures which would also benefit from use of a potassium channel activator, especially the ischemia selective activators, include liver and kidney transplants.

When administered to the mammalian organ donor or recipient or bypass patient, the dosage of potassium-channel activator should be in the range of 1-50 mg/kg. Administration to donor/recipient can be by any techniques known in the medical arts, e.g., orally, parenterally, intranasally, transdermally and the like, using known pharmaceutically acceptable formulations and delivery systems. This can be accomplished by compounding about 10 to 500 milligrams of a potassium channel activator into a pharmaceutically acceptable carrier by known techniques.

The potassium-channel activator can be present in the cardioplegic solutions in concentrations from about 3 µM to 60 µM and preferably is present in an amount ranging from 7 µM to 30 µM.

Preferred are those compounds wherein
R₁ is chloro or fluoro;
R₂ is trans-hydroxy;
R₃ and R₄ are each methyl;
R₅ is -CN or -N0₂;
R₆ is hydrogen;
R₇ is hydrogen;
R₈ is hydrogen; and,
Rg is hydrogen.

The most preferred compounds of the present invention are 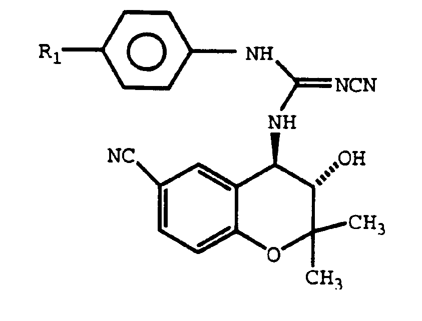 where R₁ is chloro or fluoro.

Specific embodiments of the present invention are described hereinafter in the following examples.

### Example 1

### (trans)-N"-Cyano-N-(6-cyano-3,4-dihydro-3-hydroxy-2,2-dimethyl-2H-1-benzopyran-4-yl)-N'-(4-cyanophenyl)guanidine

### A. N-Cyano-N'-4-cyanophenylthiourea

The suspension of monosodium cyanamide (4.3 g, 67.2 mmol) in absolute ethanol (170 mL) was slowly treated with 4-cyanophenylisothiocyanate (10.75 g, 67.2 mmol). The reaction was allowed to stir at room temperature for 1 hour and then heated at 75°C for 4 hours. The reaction was cooled to room temperature and the colorless solid was filtered and washed with ethanol to give the title A compound (10.0 g), m.p. >250°C.

### B. (trans)-N"-Cyano-N-(6-cyano-3,4-dihydro-3-hydroxy-2,2-dimethyl-2H-1-benzopyran-4-yl)-N'-(4-cyanophenyl)guanidine

The solution of the title A compound (1.2 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the colorless residue was crystallized from ethyl acetate to yield the title compound (0.52 g), m.p. 261-262°C. ¹H NMR (DMSO-d₆) 5 8.24 (m, 3H), 7.63 (m, 3H), 6.94 (d, J = 8.7 Hz, 1 H), 6.1 (br s, 1 H), 4.92 (t, J = 9.0 Hz, 1 H), 3.68 (br d, J = 5.2 Hz, 1 H), 1.44, 1.20 (s, 3H each). ¹³C NMR (DMSO-d₆) 6 158.7, 156.3, 145.1, 142.4, 132.9, 124.9, 124.0, 121.2, 119.1, 117.9, 116.3, 102.7, 80.4, 70.9, 51.9, 26.6, 18.6. IR (KBr) 3421.9, 2226.0, 2183.6, 1612.6, 1587.5, 1491.1, 1265.4 cm⁻¹.

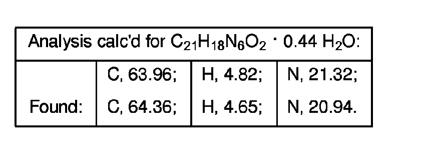

### Example 2

### (trans)-N"-Cyano-N-(6-cyano-3,4-dihydro-3-hydroxy-2,2-dimethyl-2H-1-benzopyran-4-yl)-N'-(4-methoxyphenyl)guanidine

### A. N-Cyano-N'-(4-methoxy)^{p}henylthiourea

The suspension of monosodium cyanamide (1.95 g, 30.3 mmol) in absolute ethanol (50 mL) was slowly treated with 4-methoxyphenylisothiocyanate (5.0 g, 30.3 mmol). The reaction was allowed to stir at room temperature for 1 hour and then heated at 75°C for 4 hours. The reaction was cooled to room temperature and the colorless solid was filtered and washed with ethanol to give the title A compound (5.4 g), m.p. >250°C.

### B. (trans)-N"-Cyano-N-(6-cyano-3,4-dihydro-3-hydroxy-2,2-dimethyl-2H-1-benzopyran-4-yl)-N'-(4-methoxyphenyl)guanidine

The solution of the title A compound (1.23 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the colorless residue was crystallized from ethyl acetate to yield the title compound (0.53 g), m.p. 228-229°C. ¹H NMR (DMSO-d₆) δ 9.15 (s, 1 H), 7.66 (m, 2H), 7.33 (d, J = 9.4 Hz, 3H), 6.99 (t, J = 8.8 & 8.2 Hz, 3H), 5.88 (br s, 1 H), 4.97 (t, J = 8.8 & 9.4 Hz, 1 H), 3.83 (s, 3H), 3.38 (m, H), 1.48, 1.25 (s, 3H each). ¹³C NMR (DMSO-d₆) δ 159.6. 157.1, 156.2, 132.5, 132.3, 131.6, 129.6, 126.6, 125.0, 117.8, 114.2, 102.5, 80.4, 70.6, 55.2, 51.6, 26.6, 18.5. IR (KBr) 2978.3, 2179.7, 1579.8, 1491.1, 1244.2 cm⁻¹.

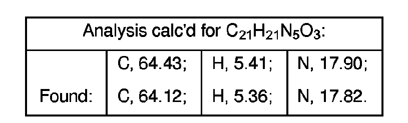

### Example 3

### (trans)-N"-Cyano-N-(6-cyano-3,4-dihydro-3-hydroxy-2,2-dimethyl-2H-1-benzopyran-4-yl)-N'-(4-nitrophenyl)guanidine

### A. N-Cyano-N'-4-nitrophenylthiourea

The suspension of monosodium cyanamide (6.4 g, 100 mmol) in absolute ethanol (170 mL) was slowly treated with (4-nitrophenyl)isothiocyanate (12.5 mL, 104.5 mmol). The reaction was allowed to stir at room temperature for 1 hour and then heated at 75°C for 4 hours. The reaction was cooled to room temperature and the colorless solid was filtered and washed with ethanol to give the title A compound (13.6 g), m.p. >250°C.

### B. (trans)-N"-Cyano-N-(6-cyano-3,4-dihydro-3-hydroxy-2,2-dimethyl-2H-1-benzopyran-4-yl)-N'-(4-nitrophenyl)guanidine

The solution of the title A compound (1.3 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the residue was flash chromatographed on silica gel with a mixture of hexane/ethyl acetate (3:7) followed by chloroformlmethanol (8:2) to give 0.6 g of product. The resulting product was triturated with ethyl acetate to yield the title compound as a colorless solid, m.p. 250-251 °C (foaming). ¹H NM R (DMSO- d₆) 8 8.23 (d, J = 8.8 Hz, 1 H), 7.88 (d, J = 8.8 Hz, 2H), 7.71 (d, J = 8.8 Hz, 2H), 7.58 (d, J = 7.6 Hz, = 2H), 7.01 (d, J = 8.8 Hz, 1 H), 6.10 (br s, 1 H), 5.01 (t, J = 8.7 & 9.4 Hz, 1 H), 3.79 (m, 1 H), 1.51, 1.28 (s, 3H each). ¹³C NMR (DMSO-d₆) δ 158.8, 156.3, 142.9, 133.3, 132.9, 124.2, 122.1, 119.1, 117.9, 105.5, 102.7, 80.4, 70.9, 51.9, 26.6, 18.6. IR (KBr) 3387.2, 2986.0, 2224.1, 2185.5, 1612.6, 1568.2, 1520.0, 1342.5, 1265.4 cm⁻¹.

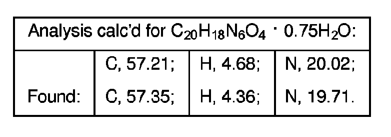

### Example 4

### (trans)-N-(4-Chlorophenyl)-N"-cyano-N-(6-cyano-3,4-dihydro-3-hydroxy-2,2-dimethyl-2H-1-benzopyran-4-yl)guanidine

### A. N-Cyano-N'-(4-chlorophenyl)thiourea

The suspension of monosodium cyanamide (1.9 g, 29.4 mmol) in absolute ethanol (50 mL) was slowly treated with 4-chlorophenylisothiocyanate (5.0 g, 29.4 mmol). The reaction was allowed to stir at room temperature for 1 hour and then heated at 75°C for 4 hours. The reaction was cooled to room temperature and the colorless solid was filtered and washed with ethanol to give the title A compound (5.4 g), m.p. >250°C.

### B. (trans)-N-(4-Chlorophenyl)-N"-cyano-N-(6-cyano-3,4-dihydro-3-hydroxy-2,2-dimethyl-2H-1-benzopyran-4-yl)guanidine

The solution of the title A compound (1.26 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the residue was purified by flash chromatography eluting with mixture of ethyl acetate/hexane (7:3). The solid was triturated with ethyl acetate to yield 0.7 g of the title compound, m.p. 216-218°C. ¹H NMR (DMSO-d₆) 5 9.43 (s, 1 H), 7.68 (m, 3H), 7.45 (m, 4H), 6.95 (d, J = 8.8 Hz, 1 H), 5.99 (br s, 1 H), 4.98 (t, J = 9.4 & 8.8 Hz, 1 H), 3.79 (m, 1 H), 1.50, 1.27 (s, 3H each). ¹³C NM R (DMSO-d₆) 6 159.1, 156.2, 136.5, 132.6, 132.5, 128.8, 125.5, 124.6, 119.0, 117.8, 116.8, 102.6, 80.4, 70.9, 51.9, 26.5, 18.5. IR (KBr) 3400.7, 2226.0, 2181.6, 1606.8, 1575.9, 1491.1, 1267.3 cm⁻¹.

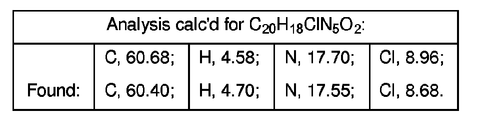

### Example 5

### (trans)-N-(2-Chlorophenyl)-N"-cyano-N-(6-cyano-3,4-dihydro-3-hydroxy-2,2-dimethyl-2H-1-benzopyran-4-yl)guanidine

### A. N-Cyano-N'-(2-chlorophenyl)thiourea

The suspension of monosodium cyanamide (1.9 g, 29.4 mmol) in absolute ethanol (50 mL) was slowly treated with 2-chlorophenylisothiocyanate (5.0 g, 29.4 mmol). The reaction was allowed to stir at room temperature for 1 hour and then heated at 75°C for 4 hours. The reaction was cooled to room temperature and the colorless solid was filtered and washed with ethanol to give the title A compound (6.0 g), m.p. 253-255°C.

### B. (trans)-N-(2-Chlorophenyl)-N"-cyano-N-(6-cyano-3,4-dihydro-3-hydroxy-2,2-dimethyl-2H-1-benzopyran-4-yl)guanidine

The solution of the title A compound (1.26 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the residue was crystallized from ethyl acetate to yield 1.1 g of the title compound, m.p. 239.240°C. ¹ H NMR (DMSO-d₆) 8 9.20 (s, 1 H), 7.63 (d, J = 8.0 Hz, 2H), 7.55 (d, J = 10.0 Hz, 2H), 7.38 (m, 2H), 6.92 (d, J = 9.0 Hz, 1H), 5.8 (br s, 1H), 4.91 (t, J = 9.4 & 8.8 Hz, 1H), 3.68 (m, 1H), 1.39, 1.17 (s, 3H each). ¹³C NMR (DMSO-d₆) δ 159.3, 156.3, 132.6, 129.8, 128.0, 124.7, 119.0, 117.9, 116.7, 102.6, 80.5, 26.6, 18.6. IR (KBr) 3432.4, 2982.6, 2225.32187.9, 1611.0, 1588.7, 1491.4, 1448.1, 1267.9 cm⁻¹.

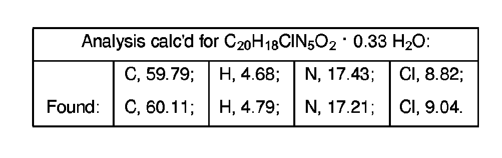

### Example 6

### (trans)-N-(3-Chlorophenyl)-N"-cyano-N-(6-cyano-3,4-dihydro-3-hydroxy-2,2-dimethyl-2H-1-benzopyran-4-yl)guanidine

### A. N-Cyano-N'-(3-chlorophenyl)thiourea

The suspension of monosodium cyanamide (1.9 g, 29.4 mmol) in absolute ethanol (50 mL) was slowly treated with 3-chlorophenylisothiocyanate (5.0 g, 29.4 mmol). The reaction was allowed to stir at room temperature for 1 hour and then heated at 75°C for 4 hours. The reaction was cooled to room temperature and the colorless solid was filtered and washed with ethanol to give the title A compound (5.4 g), m.p. 258-260°C.

### B. (trans)-N-(3-chlorophenyl)-N"-cyano-N-(6-cyano-3,4-dihydro-3-hydroxy-2,2-dimethyl-2H-1-benzopyran-4-yl)guanidine

The solution of the title A compound (1.26 g, 5.96 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated in vacuo and the residue was crystallized from ethyl acetate to yield 0.9 g of the title compound, m.p. 243-244°C. 1H NMR (DMSO-d₆) δ 9.42 (s, 1 H), 7.80 (d, J = 8.8 Hz, 1 H), 7.61 (d, J = 8.8 Hz, 2H), 7.31 (m, 3H), 6.91 (d, J = 8.8 Hz, 1 H), 5.90 (br s, 1 H), 4.98 (t, J = 9.4 & 8.8 Hz, 1 H), 3.69 (m, 1 H), 1.41, 1.18 (s, 3H each). ¹3C NMR (DMSO-d₆) δ 159.0, 156.3, 139.3, 133.1, 132.7, 130.5, 124.5, 124.3, 123.1, 121.9, 119.1, 117.9, 116.8, 102.7, 80.4, 71.0, 52.0, 26.6, 18.6. IR (KBr) 3422.4, 2980.7, 2226.5, 2181.8, 1609.3, 1575.3, 1490.1, 1385.6, 1268.1 1126.5 cm⁻¹.

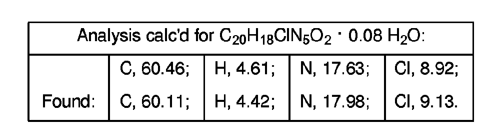

### Example 7

### (trans)-N-(4-Fluorophenyl)-N"-cyano-N-(6-cyano-3,4-dihydro-3-hydroxy-2,2-dimethyl-2H-1-benzopyran-4-yl)guanidine

### A. N-Cyano-N'-(4-fluorophenyl)thiourea

The suspension of monosodium cyanamide (2.1 g, 32.6 mmol) in absolute ethanol (50 mL) was slowly treated with 4-fluorophenylisothiocyanate (5.0 g, 32.6 mmol). The reaction was allowed to stir at room temperature for 1 hour and then heated at 75°C for 4 hours. The reaction was cooled to room temperature and the colorless solid was filtered and washed with ethanol to give the title A compound (4.1 g), m.p. >270°C.

### B. (trans)-N-(4-fluorophenyl)-N"-cyano-N-(6-cyano-3,4-dihydro-3-hydroxy-2,2-dimethyl-2H-1-benzopyran-4-yl)guanidine

The solution of the title A compound (1.15 g, 6.0 mmol) and (trans)-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem. 1986, 29, 2194) in dimethylformamide (5 mL) under argon was treated with 1-(3-dimethylaminopropyl)-2-ethylcarbodiimide hydrochloride (1.15 g, 6.0 mmol). The reaction was stirred at room temperature for 2 hours and then partitioned between 1 N hydrogen chloride and ethyl acetate. The aqueous phase was reextracted with ethyl acetate and the combined extracts were washed with water, sodium bicarbonate and brine. After drying over anhydrous magnesium sulfate, the solvent was evaporated and the residue was triturated with ethyl acetate to yield 0.8 g of the title compound, m.p. 207-208°C. ¹H NMR (DMSO-d₆) 8 9.29 (s, 1 H), 7.60 (m, 3H), 7.37 (m, 2H), 7.23 (m, 2H), 6.90 (d, J = 8.8 Hz, 1 H), 5.90 (br s, 1 H), 4.90 (t, J = 9.4 & 8.8 Hz, 1 H), 3.69 (m, 1 H), 1.40, 1.17 (s, 3H each). ¹³C NM R (DMSO-d₆) δ 159.4, 156.3, 133.6, 132.7, 132.5, 126.7, 126.6, 124.8, 119.1, 117.9, 115.8, 115.5, 102.6, 80.4, 70.8, 51.8, 26.6, 18.6. I R (KBr) 3412.9, 2980.5, 2226.9, 2179.4, 1611.4, 1585.5, 1509.9, 1490.6, 1385.4, 1268.2 cm⁻¹.

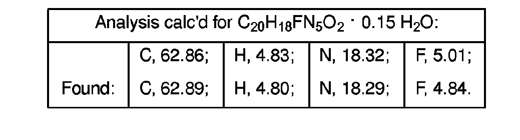

### Example 8

### (3S-trans)-N"-Cyano-N-(6-cyano-3,4-dihydro-3-hydroxy-2,2-dimethyl-2H-1-benzopyran-4-yl)-N'-(4-fluorophenyl)guanidine

### A. [3S-[3α,4β(S*)]]-N-(6-Cyano-3,4-dihydro-3-hydroxy-2,2-dimethyl-2H-1-benzopyran-4-yl)-α-hydroxybenzeneaceta- mide and

### [3R-[3α,4β(R*)]]-N-(6-Cyano-3,4-dihydro-3-hydroxy-2,2-dimethyl-2H-1-benzopyran-4-yl)-α-hydroxybenzeneacetamide

To a solution of (trans)-4-amino-3,4-dihydro-3-hydroxy-2,2-dimethyl-2H-1-benzopyran-6-carbonitrile (prepared according to Evans et al., J. Med. Chem., 1983, 26, 1582 and J. Med. Chem., 1986, 29, 2194) (1.64 g, 7.5 mmol), R(- )-mandelic acid (1.14 g, 7.5 mmol), hydroxybenzotriazole hydrate (1.0 g, 7.5 mmol) in dimethylformamide (15 ml) at 0°C was added dicyclohexylcarbodiimide (1.55 g, 7.5 mmol) at room temperature. The reaction mixture was allowed to stir at room temperature for 20 hours and then cooled in an ice bath. The solid was removed by filtration and the filtrate was concentrated in vacuo. The residue was dissolved in 5% methanol in chloroform and washed with 1 N sodium hydroxide, 1 N hydrochloric acid, brine followed by drying over anhydrous magnesium sulfate. After removing drying agent the solvent was removed in vacuo. The residue was crystallized from ethanol to give [3S-[3a,4p(S^{*})]]-N-(6-cyano-3,4-dihy- dro-3-hydroxy-2,2-dimethyl-2H-1-benzopyran-4-yl)-a-hydroxybenzeneacetamide (0.85 g) as a white solid, m.p. 235-237°C: [α_{D}]²⁵ = -94.9° (c = 1, MeOH; ¹H NMR (DMSO-d₆) δ 8.45 (d, J = 8.0 Hz, 1 H), 7.5 (m, 4 H), 7.3 (m, 2 H), 7.0 (s, 1 H), 6.88 (d, J = 8.0 Hz, 1 H), 6.2 (s, 1 H), 5.57 (d, J = 5.0 Hz, 1 H), 5.0 (s, 1 H), 4.76 (t, J = 9.0 Hz, 1 H), 3.75 (dd, J = 5.0 & 5.0 Hz, 1 H), 1.40 (s, 3 H), 1.15 (s, 3 H).

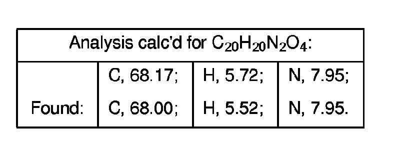

The residual material recovered from the mother liquor was purified by flash chromatography on silica gel eluting with hexane-ethyl acetate (3:7) and the product was crystallized from dichloromethane-isopropyl ether to give [3R-[3α,4β(R*)]]-N-(6-Cyano-3,4-dihydro-3-hydroxy-2,2-dimethyl-2H-1-benzopyran-4-yl)-α-hydroxy-benzeneacetamide as a white solid, m.p. 100-102°C (foaming): [aD]25 = +25.6° (c = 1, MeOH): ¹H NMR (CDCI₃) 8 7.4 (m, 5 H), 7.26 (t, J = 1.0 Hz, 1 H), 6.97 (d, J = 9.0 Hz, 1 H), 6.83 (d, J = 9.0 Hz, 1 H), 5.16 (s, 1 H), 4.98 (t, J = 9.0 Hz, 1 H), 3.8 (d, J = 5.0 Hz, 1 H), 3.55 (dd, J = 4.0 & 5.0 Hz, 1 H), 1.45 (s, 3 H), 1.2 (s, 3 H).

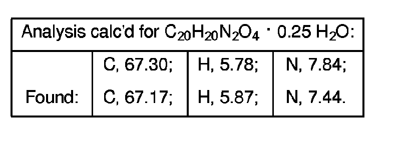

### B. (3S-trans)-4-Amino-3,4-dihyro-3-hydroxy-2,2-dimethyl-2H-1-benzopyran-6-carbonitrile

To a solution of [3S-[3α,4β(S*)]]-N-(6-cyano-3,4-dihydro-3-hydroxy-2,2-dimethyl-2H-1-benzopyran-4-yl)-α-hydroxy- benzeneacetamide, title A compound (6.09 g, 17.0 mmol) in dioxane (60 ml) was added a solution of sulfuric acid (6.0 g) in water (30 ml) at room temperature and the reaction mixture was heated at reflux temperature for 24 hours. it was then concentrated in vacuo and the residue was dissolved in ethyl acetate. The organic layer was washed with 1 N sodium hydroxide followed by water and dried over anhydrous magnesium sulfate. The solvent was evaporated to give the title B compound as an oil: ¹ H NMR (CDCI₃) 5 7.74 (s, 1 H), 7.42 (dd, J = 2.0 & 6.0 Hz, 1 H), 6.82 (d, J = 8.0 Hz, 1 H), 3.65 (d, J = 10.0 Hz, 1 H), 3.36 (d, J = 10.0 Hz, 1 H), 1.53 (s, 3 H), 1.23 (s, 3H).

### C. (3S-trans)-N"-Cyano-N-(6-cyano-3,4-dihydro-3-hydroxy-2,2-dimethyl-2H-1-benzopyran-4-yl)-N'-(4-fluorophenyl)guanidine

The solution of N-cyano-N'-(4-fluorophenyl)thiourea (1.15 g, 6.0 mmol, prepared according to Example 7, part A) and the (3S-trans)-4-amino-3,4-dihydro-3-hydroxy-2,2-dimethyl-2H-1-benzopyran-6-carbonitrile, part B of this Example, (1.0 g, 4.59 mmol) in dimethylformamide (5 mL) under argon was treated with 1-(3-dimethylaminopropyl)-2-ethylcarbodiimide hydrochloride (1.15 g, 6.0 mmol). The reaction was stirred at room temperature for 2 hours and then partitioned between 1 N hydrochloric acid and ethyl acetate. The aqueous phase was reextracted with ethyl acetate and the combined extracts were washed with water, sodium bicarbonate and brine. After drying over anhydrous magnesium sulfate, the solvent was evaporated and the residue was flash chromatographed on silica gel eluting with 20% hexanes in ethyl acetate to yield a colorless solid (0.55 g). This solid was triturated with ethyl ether to give the title compound (0.45 g), m.p. 218-219°C: ¹H NMR (DMSO-d₆) Õ 9.29 (s, 1 H), 7.60 (m, 3H), 7.37 (m, 2H), 7.23 (m, 2H), 6.90 (d, J = 8.8 Hz, 1 H), 5.90 (br s, 1 H), 4.90 (t, J = 9.4 & 8.8 Hz, 1 H), 3.69 (m, 1 H), 1.40, 1.17 (s, 3H each); ¹³C NMR (DMSO-d₆) 159.4, 156.3, 133.6, 132.7, 132.5, 126.7, 126.6, 124.8, 119.1, 117.9, 115.8, 115.5, 102.6, 80.4, 70.8, 51.8, 26.6, 18.6; IR (KBr) 3412.9, 2980.5, 2226.9, 2179.4, 1611.4, 1585.5, 1509.9, 1490.6, 1385.4, 1268.2 cm⁻¹. [α_{D}]²⁵ = -33.1 ° (c = 0.483, MeOH).

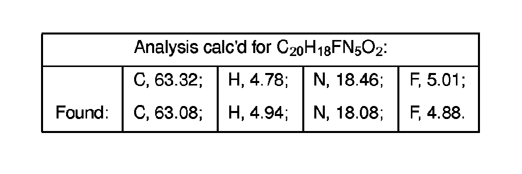

### Example 9

### (3S-trans)-N-(4-Chlorophenyl)-N"-cyano-N'-(6-cyano-3,4-dihydro-3-hydroxy-2,2-dimethyl-2H-1-benzopyran-4-yl)guanidine

The solution of N-cyano-N'-(4-chlorophenyl)-thiourea (1.26 g, 5.96 mmol, prepared according to Example 4, part A) and (3S-trans)-4-amino-3,4-dihydro-3-hydroxy-2,2-dimethyl-2H-1-benzopyran-6-carbonitrile (1.0 g, 4.59 mmol, compound of Example 8, part B) in dimethylformamide (5 mL) under argon was treated with 1-(3-dimethylaminopropyl)-2-ethylcarbodiimide hydrochloride (1.14 g, 5.96 mmol). The reaction was stirred at room temperature for 2 hours and then partitioned between 1 N hydrochloric acid and ethyl acetate. The aqueous phase was reextracted with ethyl acetate and the combined extracts were washed with water, sodium bicarbonate and brine. After drying over anhydrous magnesium sulfate, the solvent was evaporated and the residue was purified by flash chromatography eluting with a mixture of ethyl acetate/hexanes (8:2) to yield a solid (0.6 g). This solid was triturated with ethyl ether to give the title compound (0.48 g), m.p. 170-172°C: 1H NMR (DMSO-d₆) δ 9.43 (s, 1 H), 7.68 (m, 3H), 7.45 (m, 4H), 6.95 (d, J = 8.8 Hz, 1 H), 5.99 (br s, 1H), 4.98 (t, J = 9.4 & 8.8 Hz, 1H), 3.79 (m, 1H), 1.50, 1.27 (s, 3H each); ¹³C NMR (DMSO-d₆) 159.1, 156.2, 136.5, 132.6, 132.5, 128.8, 125.5, 124.6, 119.0, 117.8, 116.8, 102.6, 80.4, 70.9, 51.9, 26.5, 18.5; IR (KBr) 3400.7, 2226.0, 2181.6, 1606.8, 1575.9, 1491.1, 1267.3 cm⁻¹. [α_{D}]²⁵ = -32.9° (c = 0.492, MeOH).

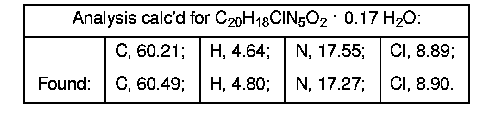

### Example 10

### (3S-trans)-N-(3-Chlorophenyl)-N"-cyano-N'-(6-cyano-3,4-dihydro-3-hydroxy-2,2-dimethyl-2H-1-benzopyran-4-yl)guanidine

The solution of N-cyano-N'-(3-chlorophenyl)thiourea (1.26 g, 5.96 mmol, prepared according to Example 6, part A) and (3S-trans)-4-amino-3,4-dihydro-3-hydroxy-2,2-dimethyl-2H-1-benzopyran-6-carbonitrile (1.0 g, 4.59 mmol, compound of Example 8, part B) in dimethylformamide (5 mL) under argon was treated with 1-(3-dimethylaminopropyl)-2-ethylcarbodiimide hydrochloride (1.17 g, 5.96 mmol). The reaction was stirred at room temperature for 2 hours and then partitioned between 1 N hydrochloric acid and ethyl acetate. The aqueous phase was reextracted with ethyl acetate and the combined extracts were washed with water, sodium bicarbonate and brine. After drying over anhydrous magnesium sulfate, the solvent was evaporated in vacuo and the residue was flash chromatographed on silica gel eluting with 20% hexanes in ethyl acetate to yield a colorless solid (1.0 g). This solid was recrystallized from ethyl acetate to give the title compound (0.36 g), m.p. 239-240°C: ¹H NMR (DMSO-d₆) δ 9.42 (s, 1 H), 7.80 (d, J = 8.8 Hz, 1 H), 7.61 (d, J = 8.8 Hz, 2H), 7.31 (m, 3H), 6.91 (d, J = 8.8 Hz, 1 H), 5.90 (br s, 1 H), 4.98 (t, J = 9.4 & 8.8 Hz, 1 H), 3.69 (m, 1 H), 1.41, 1.18 (s, 3H each); ¹3C NMR (DMSO-d₆) 159.0, 156.3, 139.3, 133.1, 132.7, 130.5, 124.5, 124.3, 123.1, 121.9, 119.1, 117.9, 116.8, 102.7, 80.4, 71.0, 52.0, 26.6, 18.6; IR (KBr) 3422.4, 2980.7, 2226.5, 2181.8, 1609.3, 1575.3, 1490.1, 1385.6, 1268.1, 1126.5 cm⁻¹. [α_{D}]²⁵ = -45.8° (c = 0.45, Dimethylformamide)

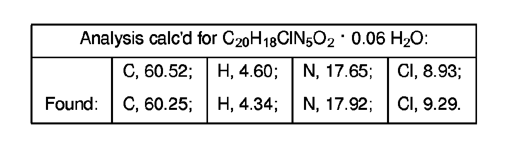

### Example 11

### trans-N"-Cyano-N-(6-cyano-3,4-dihydro-3-hydroxy-2,2-dimethyl-2H-1-benzopyran-4-yl)-N'-[4-(phenylmethoxy)phenyl)guanidine

### A. N-Cyano-N'-(4-phenylmethoxyphenyl)thiourea

The suspension of monosodium cyanamide (1.33 g, 20.7 mmol) in absolute ethanol (50 mL) was slowly treated with 4-phenylmethoxyphenylisothiocyanate (5.0 g, 20.7 mmol). The reaction was allowed to stir at room temperature for 1 hour and then heated at 75°C for 4 hours. The reaction was cooled to room temperature and the colorless solid was filtered and washed with ethanol to give the title A compound (4.0 g), m.p. >270°C.

### B. trans-N"-Cyano-N-(6-cyano-3,4-dihydro-3-hydroxy-2,2-dimethyl-2H-1-benzopyran-4-yl)-N'-[4-(phenylmethoxy)phenyl)guanidine

The solution of the title A compound (1.68 g, 6.0 mmol) and trans-4-amino-3,4-dihydro-3-hydroxy-2,2-dimethyl-2H-1-benzopyran-6-carbonitrile (1.0 g, 4.59 mmol, prepared according to Evans et al., J. Med. Chem., 1983, 26, 1582 and J. Med. Chem., 1986, 29, 2194) in dimethylformamide (5 mL) under argon was treated with 1-(3-dimethylaminopropyl)-2-ethylcarbodiimide hydrochloride (1.15 g, 6.0 mmol). The reaction was stirred at room temperature for 2 hours and then partitioned between 10% citric acid and ethyl acetate and the solid was separated out. The aqueous phase was reextracted with ethyl acetate and the combined extracts were washed with water, sodium bicarbonate and brine. After drying over anhydrous magnesium sulfate, the solvent was evaporated and the residue was combined with previous solid and crystallized from hot ethyl acetate to give the title compound as a colorless solid (1.1 g), m.p. 229-230°C: ¹H NMR (DMSO-d₆) 5 9.13 (s, 1 H), 7.62 (m, 2H), 7.37 (m, 6H), 7.24 (d, J = 8.8 Hz, 2H), 7.0 (d, J = 8.8 Hz, 2H), 6.89 (d, J = 8.2 Hz, 1 H), 5.85 (br s, 1 H), 5.1 (s, 2H), 4.90 (t, J = 9.0 Hz, 1 H), 3.69 (m, 1 H), 1.40, 1.16 (s, 3H each); ¹³C NMR (DMSO- d₆) 159.6, 156.3, 137.0, 132.6, 132.4, 128.5, 127.9, 127.7, 126.6, 125.0, 119.1, 117.8, 117.3, 115.2, 102.6, 80.4, 70.6, 69.4, 51.6, 26.6, 18.6; IR (KBr) 2978.0, 2936.0, 2226.3, 2180.7, 1610.0, 1581.3, 1510.9, 1489.7, 1267.5, 1238.5 cm⁻¹.

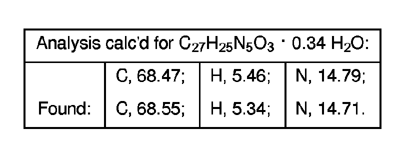

### Example 12

### trans-N"-Cyano-N-(6-cyano-3,4-dihydro-3-hydroxy-2,2-dimethyl-2H-1-benzopyran-4-yl)-N'-(4-hydroxyphenyl)guanidine

To a solution of the title compound from Example 11 (0.7 g, 1.5 mmol) in ethanol (70 mL) was added (10%) palladium on carbon (0.1 g). It was then treated with hydrogen in a balloon and heated at 60°C for 2 hours. The reaction was filtered through a pad of celite, the filtrate was washed with ethanol and concentrated in vacuo to give the title compound as a colorless solid (0.5 g), m.p. 171-173°C: ¹H NMR (DMSO-d₆) 6 9.40 (s, 1 H), 9.03 (s, 1 H), 7.58 (d, J = 8.2 Hz, 1 H), 7.51 (s, 2H), 7.2 (s, 1 H), 7.11 (d, J = 8.8 Hz, 2H), 6.89 (d, J = 8.2 Hz, 1 H), 6.73 (d, J = 8.8 Hz, 2H), 5.85 (br s, 1 H), 4.87 (t, J = 9.0 Hz, 1H), 3.71 (m, 1H), 1.38, 1.15 (s, 3H each); ¹3C NMR (DMSO-d₆) 159.6, 156.3, 132.6, 132.4, 127.0, 126.6, 119.1, 117.8, 117.3, 115.6, 102.6, 80.4, 79.4, 70.6, 51.6, 26.7, 18.6; IR (KBr) 3485.6, 2986.0, 2941.6, 2226.0, 1585.6, 1514.2, 1491.1, 1307.8, 1271.2, 1128.4 cm⁻¹.

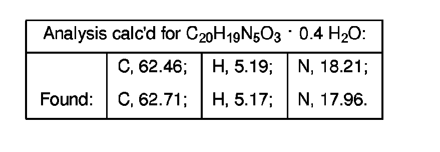

### Example 13

### (3S-trans)-N-(3,4-Dichlorophenyl)-N"-cyano-N'-(6-cyano-3,4-dihydro-3-hydroxy-2,2-dimethyl-2H-1-benzopyran-4-yl)guanidine

### A. N-Cyano-N'-(3,4-dichlorophenyl)thiourea

The suspension of monosodium cyanamide (1.6 g, 24.5 mmol) in absolute ethanol (50 mL) was slowly treated with 3,4-dichlorophenylisothiocyanate (5.0 g, 24.5 mmol). The reaction was allowed to stir at room temperature for 1 hour and then heated at 75°C for 4 hours. The reaction was cooled to room temperature and the colorless solid was filtered and washed with ethanol to give the title A compound (5.0 g) as a colorless solid.

### B. (3S-trans)-N-(3,4-Dichlorophenyl)-N"-cyano-N'-(6-cyano-3,4-dihydro-3-hydroxy-2,2-dimethyl-2H-1-benzopyran-4-yl)guanidine

The solution of the title A compound (1.47 g, 6.0 mmol) and (3S-trans)-4-amino-3,4-dihydro-3-hydroxy-2,2-dimethyl-2H-1-benzopyran-6-carbonitrile (1.0 g, 4.6 mmol, compound of Example 8, part B) in dimethylformamide (10 mL) under argon was treated with 1-(3-dimethylaminopropyl)-2-ethylcarbodiimide hydrochloride (1.13 g, 6.0 mmol). The reaction was stirred at room temperature for 2 hours and then partitioned between pH 4 buffer and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography (ethyl acetate:hexanes/7:3) to yield a colorless solid (0.6 g). The solid was triturated with ethyl ether to give the title compound (0.5 g), m.p. 168-170°C: 1H NMR (CDCl₃) 5 9.30 (s, 1 H), 7.64 (s, 1 H), 7.58 (d, J = 2.4 Hz, 1 H), 7.40 (m, 2H), 7.30 (dd, J = 2.3 & 2.9, 1 H), 6.85 (d, J = 8.8 Hz, 1 H), 4.97 (m, 1 H), 3.70 (d, J = 10.0 Hz, 1 H), 1.49, 1.26 (s, 3H each); ¹3C NMR (CDCI₃) 158.6, 155.8, 136.4, 131.9, 131.4, 129.7, 118.1, 117.3, 102.6, 79.6, 51.8, 25.8, 17.8; IR (KBr) 3398, 2980, 2225, 2183, 1610, 1581, 1489, 1371 cm⁻¹. [a_{D]}²⁵ = -35.37° (c = 0.458, dimethylformamide).

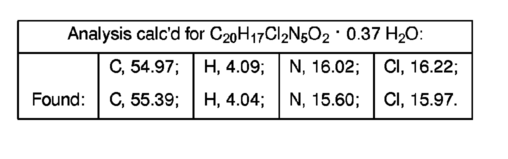

### Example 14

### (3S-trans)-N"-Cyano-N-(6-cyano-3,4-dihydro-3-hydroxy-2,2-dimethyl-2H-1-benzopyran-4-yl)-N'-[4-(trifluorome- th^{y}l)^{p}henyl)l^{g}uanidine

### A. N-Cyano-N'-(4-trifluoromethylphenyl)thiourea

The suspension of monosodium cyanamide (0.63 g, 9.8 mmol) in absolute ethanol (50 mL) was slowly treated with 4-trifluoromethylphenylisothiocyanate (2.0 g, 9.8 mmol). The reaction was allowed to stir at room temperature for 1 hour and then heated at 75°C for 4 hours. The reaction was cooled to room temperature and the colorless solid was filtered and washed with ethanol to give the title compound (2.0 g) as a colorless solid.

### B. (3S-trans)-N"-Cyano-N-(6-cyano-3,4-dihydro-3-hydroxy-2,2-dimethyl-2H-1-benzopyran-4-yl)-N'-[4-(trifluorome- th^{y}l)^{p}henyl)l^{g}uanidine

The solution of the title A compound (1.3 g, 5.3 mmol) and (3S-trans)-4-amino-3,4-dihydro-3-hydroxy-2,2-dimethyl-2H-1-benzopyran-6-carbonitrile (0.83 g, 3.8 mmol, prepared according to Example 8, part B) in dimethylformamide (10 mL) under argon was treated with 1-(3-dimethylaminopropyl)-2-ethylcarbodiimide hydrochloride (1.1 g, 5.7 mmol). The reaction was stirred at room temperature for 2 hours and then partitioned between pH 4 buffer and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the resiude was purified by flash chromatography eluting with a mixture of ethyl acetate/hexanes (7:3). The solid was triturated with ethyl ether to give the title compound (0.45 g), m.p. 209-210°C: ¹H NMR (CDCI₃) δ 9.41 (s, 1 H), 7.60 (m, 6H), 6.85 (d, J = 8.8 Hz, 1 H), 4.99 (m, 1 H), 3.74 (d, J = 9.4 Hz, 1 H), 1.50, 1.28 (s, 3H each); ¹³C NM R (CDCI₃) 158.7, 156.0, 140.4, 132.1, 125.5, 123.9, 121.9, 118.3, 117.5, 102.8, 79.8, 52.1, 25.9, 18.0; IR (KBr) 3403, 2226, 2184, 1588, 1491, 1325, 1126, 1069 cm⁻¹. [α_{D}]²⁵ = -40.2 (c = 0.567, MeOH).

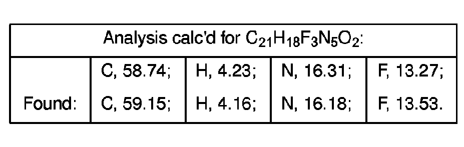

### Bioavailability and Pharmacokinetics in Rats and Dogs

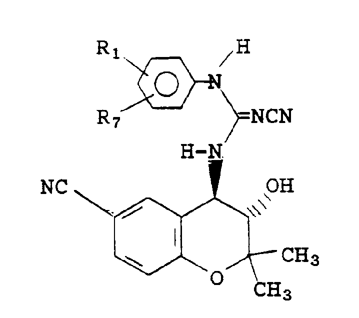

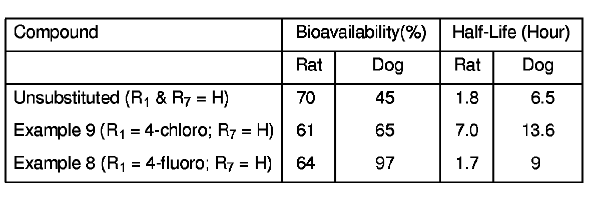

### Example 15

Using the methodology below, bioavailability and duration of action were determined for two substituted phenyl compounds of the present invention versus an unsubstituted phenyl compound.

### Studies in Dogs

Single 25-wmol/kg doses of a compound of Example 9 were administered intravenously as a solution in 100% polyethylene glycol (PEG) and orally as a suspension in PEG in gelatin capsules to three dogs with a 15 day washout period between doses. Similarly, 25-wmol/kg doses of a compound of Example 8 were administered intravenously as a solution in 50% PEG/water and orally as a suspension in PEG in gelatin capsules to two other dogs with a 19-day washout period between doses. The unsubstituted compound (20 µmol/kg) was administered both orally and intravenously as a solution in 50% PEG/water to two dogs with a one-week washout period between doses. Plasma samples were obtained at various times after dosing and analyzed by high pressure liquid chromatography (HPLC) methods for parent drug and possible metabolites.

### Studies in Rats

In studies of the pharmacokinetics and disposition of the potassium channel activators in rats, the compound of interest was administered as a solution in 50% PEG/water. Each compound was administered intravenously (N=3) and orally (N=3) at a dose of 53 umol/kg (unsubstituted compound), 45 umol/kg (compound of Example 9) or 43 umol/kg (compound of Example 8). Plasma and urine samples were obtained at various times after dosing and analyzed by HPLC methods for parent drug and possible metabolites.

### Example 16

### (3S-trans)-N"-Cyano-N-(6-cyano-3,4-dihydro-3-hydroxy-2,2-dimethyl-2H-1-benzopyran-4-yl)-N'-(4-nitrophenyl)guanidine

### A. N-Cyano-N'-(4-nitrophenyl)thiourea

The suspension of monosodium cyanamide (2.7 g, 42.4 mmol) in absolute ethanol (50 mL) was slowly treated with 4-nitrophenylisothiocyanate (5.0 g, 42.4 mmol). The reaction was allowed to stir at room temperature for 1 hour and then heated at 75°C for 12 hours. The reaction was cooled to room temperature and concentrated in vacuo and triturated with ethyl ether to give the title A compound (5.0 g).

### B. (3S-trans)-N"-Cyano-N-(6-cyano-3,4-dihydro-3-hydroxy-2,2-dimethyl-2H-1-benzopyran-4-yl)-N'-(4-nitrophenyl)guanidine

The solution of the title A compound (1.53 g, 6.9 mmol) and (3S-trans)-4-amino-3,4-dihydro-3-hydroxy-2,2-dimethyl-2H-1-benzopyran-6-carbonitrile (prepared from Example 8, part B) (1.0 g, 4.6 mmol) in dimethylformamide (5 mL) under argon was treated with 1-(3-dimethylaminopropyl)-2-ethylcarbodiimide hydrochloride (1.13 g, 6.0 mmol). The reaction was stirred at room temperature for 2 hours and then partitioned between 1 N hydrochloric acid solution and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography eluting with a mixture of dichloromethanelacetone (9:1) to yield 0.8 g of the crude product. The solid was triturated with ethyl ether to give the title compound, m.p. 165-170°C (foaming): ¹H NMR (DMSO-d₆) δ 8.22 (m, 3H), 7.62 (m, 4H), 6.94 (d, J = 8.8 Hz, 1 H), 6.0 (s, 1 H), 4.97 (t, J = 8.8 & 9.4 Hz, 1 H), 3.73 (m, 1H), 1.45, 1.21 (s, 3H each); ¹3C NMR (DMSO-d₆) 158.8 156.3, 145.2, 142.4, 132.9, 124.9, 124.0, 121.2, 119.0, 118.0, 116.3, 102.8, 80.5, 71.2, 52.4, 26.5, 18.6; IR (KBr) 3428, 2226, 2182, 1613, 1593, 1564, 1491, 1339, 1269 cm⁻¹.

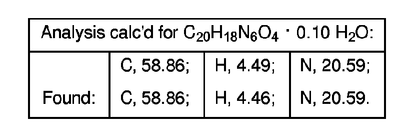

[α_{D}]²⁵ = -87.0° (c = 0.866, MeOH).

### Example 17

### (3S-trans)-N-(3-Nitrophenyl)-N"-cyano-N'-(6-cyano-3,4-dihydro-3-hydroxy-2,2-dimethyl-2H-1-benzopyran-4-yl)guanidine

### A. N-Cyano-N'-(3-nitrophenyl)thiourea

The suspension of monosodium cyanamide (2.7 g, 42.4 mmol) in absolute ethanol (50 mL) was slowly treated with 3-nitrophenylisothiocyanate (5.0 g, 42.4 mmol). The reaction was allowed to stir at room temperature for 1 hour and then heated at 75°C for 12 hours. The reaction was cooled to room temperature and concentrated in vacuo and triturated with ethyl ether to give the title A compound (5.0 g).

### B. (3S-trans)-N-(3-Nitrophenyl)-N"-cyano-N'-(6-cyano-3,4-dihydro-3-hydroxy-2,2-dimethyl-2H-1-benzopyran-4-yl)guanidine

The solution of the title A compound (1.53 g, 6.9 mmol) and (3S-trans)-4-amino-3,4-dihydro-3-hydroxy-2,2-dimethyl-2H-1-benzopyran-6-carbonitrile (prepared from Example 8, part B) (1.0 g, 4.6 mmol) in dimethylformamide (5 mL) under argon was treated with (1-(3-dimethylaminopropyl)-2-ethylcarbodiimide hydrochloride (1.13 g, 6.0 mmol). The reaction was stirred at room temperature for 2 hours and then partitioned between 1 N hydrochloric acid solution and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography eluting with a mixture of ethyl acetate/hexanes (6:4) to yield 0.5 g of the crude solid. The solid was triturated with ethyl ether to give the title compound (0.5 g), m.p. 214-216°C; ¹H NMR (DMSO- d₆) δ 8.65 (s, 1 H), 8.24 (s, 1 H), 7.95 (m, 2H), 7.73 (m, 2H), 7.61 (m, 2H), 6.92 (d, J = 8.8 Hz, 1 H), 4.93 (m, 1 H), 3.71 (m, 1H), 1.42, 1.20 (s, 3H each); ¹³C NMR (DMSO-d₆) 159.2, 156.6, 148.3, 139.6, 133.1, 130.5, 129.7, 124.6, 119.3, 119.1, 118.2, 117.9, 116.9, 103.0, 80.7, 71.5, 52.5, 26.9, 19.0; IR (KBr) 3374, 2980, 2228, 2184, 1610, 1530, 1489, 1454, 1348, 1267 cm⁻¹.

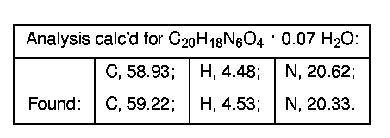

[a_{D]}²⁵ = -28.0° (c = 0.642, DMF).

### Example 18

### (3S-trans)-N-(3-Trifluoromethylphenyl)-N"-cyano-N'-(6-cyano-3.4-dihydro-3-hydroxy-2.2-dimethyl-2H-1-benzopyran-4-yl)guanidine

### A. N-Cyano-N'-(3-trifluoromethylphenyl)thiourea

The suspension of monosodium cyanide (1.57 g, 24.5 mmol) in absolute ethanol (50 mL) was slowly treated with 3-trifluoromethylphenylisothiocyanate (5.0 g, 24.5 mmol). The reaction was allowed to stir at room temperature for 1 hour and then heated at 75°C for 10 hours. The reaction was cooled to room temperature and concentrated in vacuo to give the title A compound (5.0 g).

### B. (3S-trans)-N-(3-Trifluoromethylphenyl)-N"-cyano-N'-(6-cyano-3,4-dihydro-3-hydroxy-2,2-dimethyl-2H-1-benzopyran-4-yl)guanidine

The solution of the title A compound (1.7 g, 6.9 mmol) and (3S-trans)-4-amino-3,4-dihydro-3-hydroxy-2,2-dimethyl-2H-1-benzopyran-6-carbonitrile (prepared from Example 8, part B) (1.0 g, 4.6 mmol) in dimethylformamide (5 mL) under argon was treated with 1-(3-dimethylaminopropyl)-2-ethylcarbodiimide hydrochloride (1.13 g, 6.0 mmol). The reaction was stirred at room temperature for 2 hours and then partitioned between 1 N hydrochloric acid solution and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography eluting with a mixture of dichloromethane/acetone (9:1) to yield 0.8 g of the crude solid. The solid was triturated with ethyl ether to give the title compound (0.6 g), m.p. 205-208°C (foaming): ¹ H NMR (DMSO-d₆) 8 9.60 (s, 1 H), 7.90 (d,m J = 8.8 Hz, 1 H), 7.62 (m, 6H), 6.92 (d, J = 8.2 Hz, 1 H), 6.0 (s, 1 H), 4.94 (m, 1H), 3.71 (m, 1H), 1.42, 1.19 (s, 3H each); ¹3C NMR (DMSO-d₆) 159.0, 138.8, 132.8, 130.0, 129.9, 129.4, 127.0, 126.0, 124.4, 122.0, 120.8, 119.7, 119.0, 117.9, 116.7, 102.7, 80.4, 71.2, 52.1, 26.6, 18.6; IR (KBr) 3418, 2226, 2182, 1588, 1491, 1333, 1128 cm⁻¹.

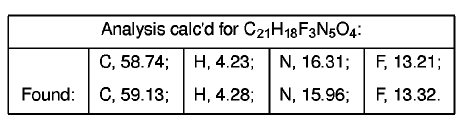

IaD125 = -38.0° (c = 0.908, DMF).

### Example 19

### (3S-trans)-N"-Cyano-N-(6-cyano-3,4-dihydro-3-hydroxy-2,2-dimethyl-2H-1-benzopyran-4-yl)-N'-(3-methylphenyl)guanidine

### A. N-Cyano-N'-(3-methylphenyl)thiourea

The suspension of monosodium cyanamide (2.11 g, 33 mmol) in absolute ethanol (50 mL) was slowly treated with 3-methylphenylisothiocyanate (5.0 g, 33 mmol). The reaction was allowed to stir at room temperature for 1 hour and then heated at 75°C for 8 hours. The reaction was cooled to room temperature and concentrated in vacuo and triturated with ethyl ether to give the title A compound (4.5 g)

### B. (3S-trans)-N"-Cyano-N-(6-cyano-3,4-dihydro-3-hydroxy-2,2-dimethyl-2H-1-benzopyran-4-yl)-N'-(3-methylphe- n^{y}l)auanidine

The solution of the title A compound (1.3 g, 6.9 mmol) and (3S-trans)-4-amino-3,4-dihydro-3-hydroxy-2,2-dimethyl-2H-1-benzopyran-6-carbonitrile (prepared from Example 8, part B) (1.0 g, 4.6 mmol) in dimethylformamide (5 mL) under argon was treated with 1-(3-dimethylaminopropyl)-2-ethylcarbodiimide hydrochloride (1.13 g, 6.0 mmol). The reaction was stirred at room temperature for 2 hours and then partitioned between 1 N hydrochloric acid solution and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography eluting with a mixture of dichloromethane/acetone (9:1) to yield 0.8g of crude solid. The solid was triturated with ethyl ether to give the title compound, m.p. 213-214°C (foaming): ¹H NMR (DMSO-d₆) δ 9.2 (s, 1 H), 7.62 (m, 3H), 7.23 (m, 3H), 6.97 (d, J = 7.0 Hz, 1 H), 6.90 (d, J = 8.8 Hz, 1 H), 5.9 (s, 1 H), 4.92 (t, J = 8.8 & 9.3 Hz, 1 H), 3.70 (m, 1 H), 2.3, 1.45, 1.21 (s, 3H each); ¹3C NMR (DMSO-d₆) 159.5, 156.6, 138.6, 137.6, 132.9, 132.8, 129.1, 125.8, 125.1, 124.6, 121.1, 119.3, 118.1, 117.4, 102.9, 80.7, 71.2, 52.1, 26.9, 21.3, 18.9; IR (KBr) 3391, 2226, 2182, 1582, 1489, 1371, 1267 cm⁻¹.

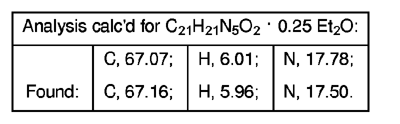

[α_{D}]²⁵ = -37.1° (c = 0.542, DMF).

### Example 20

### (3S-trans)-N"-Cyano-N-(6-cyano-3,4-dihydro-3-hydroxy-2,2-dimethyl-2H-1-benzopyran-4-yl)-N'-(4-methylphenyl)guanidine

### A. N-Cyano-N'-(4-methylphenyl)thiourea

The suspension of monosodium cyanamide (2.11 g, 33 mmol) in absolute ethanol (50 mL) was slowly treated with 4-methylphenylisothiocyanate (5.0 g, 33 mmol). The reaction was heated under reflux temperature for 16 hours. The reaction was cooled to room temperature, concentrated in vacuo and triturated with ethyl ether to give the title A compound (5.0 g).

### B. (3S-trans)-N"-Cyano-N-(6-cyano-3,4-dihydro-3-hydroxy-2,2-dimethyl-2H-1-benzopyran-4-yl)-N'-(4-methylphe- n^{y}l)auanidine

The solution of the title A compound (1.3 g, 6.9 mmol) and (3S-trans)-4-amino-3,4-dihydro-3-hydroxy-2,2-dimethyl-2H-1-benzopyran-6-carbonitrile (prepared from Example 8, part B) (1.0 g, 4.6 mmol) in dimethylformamide (5 mL) under argon was treated with 1-(3-dimethylaminopropyl)-2-ethylcarbodiimide hydrochloride (1.13 g, 6.0 mmol). The reaction was stirred at room temperature for 2 hours and then partitioned between 1 N hydrochloric acid solution and ethyl acetate. The aqueous phase was reextracted with ethyl acetate and the combined extracts were washed with water (4 x 200 ml), sodium bicarbonate and brine. After drying over anhydrous magnesium sulfate, the solvent was evaporated and the residue was purified by flash chromatography eluting with a mixture of 15% acetone in dichloromethane to yield 0.5 g of the crude solid. The solid was triturated with ethyl ether to give the title compound, m.p. 146-150°C (foaming): ¹H NM R (DMSO-d₆) δ 9.39 (s, 1 H), 7.74 (m, 3H), 7.40 (dd, J = 8.2 Hz, 3H), 7.08 (d, J = 8.8 Hz, 1 H), 6.1 (s, 1 H), 5.09 (m, 1 H), 3.90 (m, 1 H), 2.47, 1.59, 1.28 (s, 3H each); ¹3C NMR (DMSO-d₆) 159.3, 156.2, 134.6, 134.1, 132.6, 132.4, 129.5, 129.3, 124.9, 124.2, 119.1, 117.1, 102.5, 80.4, 70.7, 51.7, 26.6, 20.4, 18.5; IR (KBr) 3401, 2224, 2182, 1576, 1489, 1283 cm⁻¹.

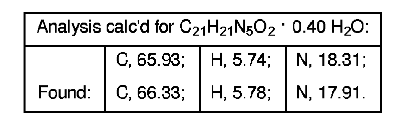

[α_{D}]²⁵ = -16.5° (c = 0.725, MeOH).

### Example 21

In this example, rat aorta and rat heart data are presented for the compound of Examples 1-20. The methodologie are described immediately below. Briefly, the rat aorta data is compared to the potent vasodilator cromakalim to illustrate the selectivity (lack of vasodilating effect in normal tissue) of the present compound. The rat heart methology involves a glabally ischemic rat heart model which is believed to be a reliable indicator of protection for organ surgery procedures. This is because the laboratory-induced isolation and ischemic event, including perfusion with a cardioplegic solution, reasonably duplicates the environment and conditions for the heart during bypass and transplant. Some compounds were tested by measuring the percent decrease in lactate dehydrogenase (LDH) release; other were tested by measuring the increase in time to contracture.

### Rat Aorta Methodology

Following sacrifice, the thoracic aorta was removed from male Wistar Kyoto rats and placed in cold physiological salt solution (PSS) containing (in mM): 118.4 NaCl, 4.7 KCI, 1.2 KH₂PO₄, 1.2 MgS0₄, 2.5 CaC1₂, 25.0 NaHC0₃ and 11.7 glucose. Rings were cut from each aorta and the endothelium was mechanically removed. The rings were individually mounted for isometric force recording and suspended in individual chambers containing PSS at 37°C aerated with 95% 0₂/5% C0₂ (pH 7.4). During the equilibration period, the rings were stretched to 2 g and stimulated with 24.7 mM KCI several times to determine contractibility.

Following the equilibration period, propranolol (1 µM) was added to each chamber to block beta-adrenoceptors. Rings were contracted with 0.3 µM methoxamine, then a cumulative concentration relaxation curve was obtained for the test compound. After the final bath concentration was attained, "reversal" of the relaxation was attempted (in the continued presence of the test compound) by addition of sufficient quantities of a 4 M KCI solution to obtain a final bath concentration of 60 mM KCI.

Data are presented as mean ± SEM for at least 4 rings from different animals. The IC₅₀ values were determined from a quadratic fit to the logit transformation of the concentration relaxation curves. Concentrated stock solutions of test compounds were prepared daily in water or DMSO as appropriate.

### Rat Heart (% decrease in LDH)

Preparation of the Isolated Perfused Hearts. Male Sprague-Dawley rats (450-550 g) were used in all experiments. The rats were anesthetized using 30 mg/kg sodium pentobarbital (i.p.). They were intubated and then treated with i.v., heparin (1000 U/kg). While being mechanically ventilated, their hearts were perfused in situ via retrograde cannulation of the aorta. The hearts were then excised and quickly moved to a Langendorff apparatus where they were perfused with Krebs-Henseleit bicarbonate buffer (112 mM NaC1₂, 25 mM NaHC0₃, 5 mM KCI, 1.2 mM MgS0₄, 1 mM KH₂PO₄, 1.25 mM CaCl2, 11.5 mM dextrose, and 2 mM pyruvate bubbled with 95% 0₂ - 5% C0₂) at a constant pressure (75 mm Hg). A water filled latex balloon attached to a metal cannula was then inserted into the left ventricle and connected to a Statham pressure transducer for measurement of left ventricular pressure. The hearts were allowed to equilibrate for 15 minutes at which time end diastolic pressure (EDP) was adjusted to 5 mm Hg and this was maintained for 5 minutes. Pre-ischemia or pre-drug function, heart rate and coronary flow (extra-corporeal electromagnetic flow probe, Carolina Medical Electronics, King, N.C.) were then measured. Cardiac function was determined using the double product of heart rate (HR) x left ventricular developed pressure (LVDP) divided by 1000. Cardiac temperature was maintained throughout the experiment by submerging the hearts in 37°C buffer which was allowed to accumulate in a stoppered, heated chamber.

Experimental Protocol. Once the baseline measurements were taken, the hearts were treated with the listed compounds or with vehicle buffer (0.01% DMSO, n = 7). All of these hearts were treated with their respective drugs or vehicle for ten minutes. At this time, post-drug cardiac function and flow were measured and then the hearts were made globally ischemic by shutting off the buffer perfusion. The ischemia was maintained for 25 minutes, the hearts were then reperfused with nondrug treated buffer. Reperfusion was maintained for a total of 30 minutes and at this time reperfusion function and flow were again determined. The results are summarized in the TABLE below.

### Rat Heart Time to Contracture (EC₂₅)

Time to contracture; EC25; concentration increasing time to contracture by 25%; or increase in time to contracture at 10 gM.

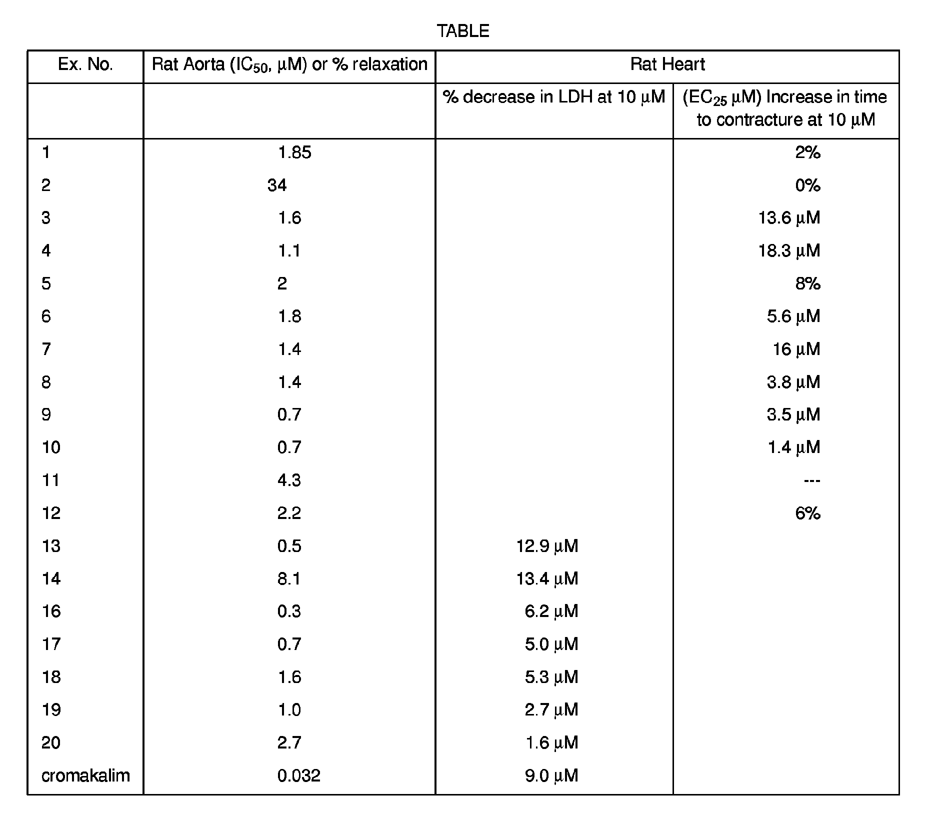

## Claims (Claims for the following Contracting State(s) : AT, BE, CH, LI, DE, DK, FR, GB, IT, LU, MC, NL, PT, SE)

1. A compound of the formula 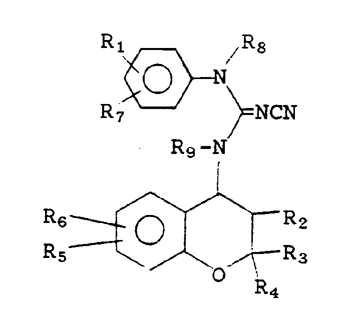
R₁ and R₇ are each independently hydrogen cyano, alkoxy, nitro, halo, hydroxy, haloalkyl, or benzyloxy, with the proviso that at least one of R₁ and R₇ is other than hydrogen;
R₂ is hydrogen, hydroxy, or 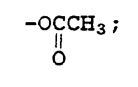
R₃ and R₄ are each independently hydrogen, alkyl or arylalkyl, or R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO_{z}, -COR, -COOR, -CONHR, -CONR₂, -CF₃, S-alkyl, -SOalkyl, -SO₂alkyl, 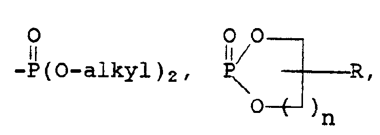 halogen, amino, substituted amino, O-alkyl, OCF₃, OCH₂CF₃, -OCOalkyl, -OCONRalkyl, -NRCOalkyl, NRCOOalkyl and NRCONR₂, wherein R in each of the above groups can be hydrogen, alkyl, haloalkyl, aryl, arylalkyl, cycloalkyl, or (cycloalkyl)alkyl;
R₆ is selected from H, alkyl, OH, O-alkyl, amino, substituted amino, NHCOR (wherein R is as defined above), CN, and NO_{z};
R₈ is selected from hydrogen and alkyl;
Rg is selected from hydrogen, alkyl, alkenyl, aryl, arylalkyl, cycloalkyl and cycloalkylalkyl; and
n is 1,2 or 3;
wherein:
"alkyl", "alkenyl", "alkynyl" and "alkoxy" refer to such groups having up to eight carbons;
"cycloalkyl" refers to such groups having three to seven carbons;
"halo" and "halogen" refer to chloro, bromo and fluoro;
"aryl" refers to phenyl, 1-naphthyl, 2-naphthyl; mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons, -CF₃, -OCHF₂, 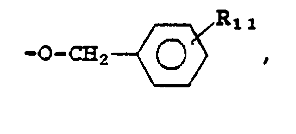 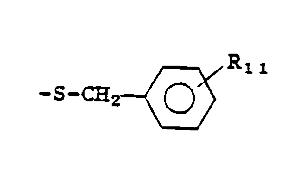
(wherein R₁₁ is hydrogen,
alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl; and di-substituted phenyl, 1-naphthyl, 2-naphthyl wherein said substituents are selected from methyl, methoxy, methylthio, halo, CF₃, nitro, amino, and OCHF₂; and
"substituted amino" refers to a group of the formula -NZ₁Z₂ wherein Z₁ is hydrogen, alkyl, cycloalkyl, aryl, arylalkyl or cycloalkylalkyl, and Z₂ is alkyl, cycloalkyl, aryl, arylalkyl or cycloalkylalkyl, or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy

2. A compound in accordance with claim 1 wherein
R₁ is chloro or fluoro;
R₂ is trans-hydroxy;
R₃ and R₄ are each methyl;
R₅ is -CN or -NO2;
R₆ is hydrogen;
R₇ is hydrogen;
R₈ is hydrogen; and,
Rg is hydrogen.

3. A compound in accordance with claim 1 having the formula 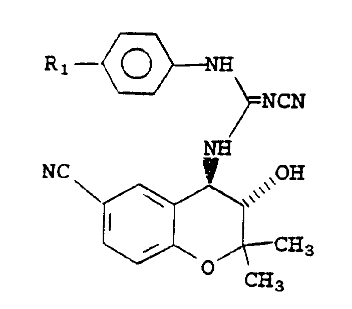 where R₁ is fluoro or chloro.

4. A compound in accordance with claim 1 which is (trans)-N"-cyano-N-(6-cyano-3,4-dihydro-3-hydroxy-2,2-dimethyl-2H-1-benzopyran-4-yl)-N'-(4-cyanophenyl)guanidine.

5. A compound in accordance with claim 1 which is (trans)-N"-cyano-N-(6-cyano-3,4-dihydro-3-hydroxy-2,2-dimethyl-2H-1-benzopyran-4-yl)-N'-(4-methoxyphenyl)guanidine.

6. A compound in accordance with claim 1 which is (trans)-N"-cyano-N-(6-cyano-3,4-dihydro-3-hydroxy-2,2-dimethyl-2H-1-benzopyran-4-yl)-N'-(4-nitrophenyl)guanidine.

7. A compound in accordance with claim 1 which is (trans)-N-(4-chlorophenyl)-N"-cyano-N-(6-cyano-3,4-dihydro-3-hydroxy-2,2-dimethyl-2H-1-benzopyran-4-yl)guanidine.

8. A compound in accordance with claim 1 which is (trans)-N-(2-chlorophenyl)-N°-cyano-N-(6-cyano-3,4-dihydro-3-hydroxy-2,2-dimethyl-2H-1-benzopyran-4-yl)guanidine.

9. A compound in accordance with claim 1 which is (trans)-N-(3-chlorophenyl)-N"-cyano-N-(6-cyano-3,4-dihydro-3-hydroxy-2,2-dimethyl-2H-1-benzopyran-4-yl)-guanidine.

10. A compound in accordance with claim 1 which is (trans)-N-(4-fluorophenyl)-N"-cyano-N-(6-cyano-3,4-dihydro-3-hydroxy-2,2-dimethyl-2H-1-benzopyran-4-yl)guanidine.

11. A compound in accordance with claim 1 which is (3S-trans)-N"-cyano-N-(6-cyano-3,4-dihydro-3-hydroxy-2,2-dimethyl-2H-1-benzopyran-4-yl)-N'-(4-fluorophenyl)guanidine.

12. A compound in accordance with claim 1 which is (3S-trans)-N-(4-chlorophenyl)-N'-(cyano-N'-(6-cyano-3,4-dihydro-3-hydroxy-2,2-dimethyl-2H-1-benzopyran-4-yl)guanidine.

13. A compound in accordance with claim 1 which is (3S-frans)-N-(3-chlorophenyl)-N"-cyano-N'-(6-cyano-3,4-dihydro-3-hydroxy-2,2-dimethyl-2H-1-benzopyran-4-yl)guanidine.

14. A compound in accordance with claim 1 which is trans-N"-cyano-N-(6-cyano-3,4-dihydro-3-hydroxy-2,2-dimethyl-2H-1-benzopyran-4-yl)-N'-[4-(phenylmethony)phenyl)guanidine.

15. A compound in accordance with claim 1 which is trans-N"-cyano-N-(6-cyano-3,4-dihydro-3-hydroxy-2,2-dimethyl-2H-1-benzopyran-4-yl)-N'-(4-hydroxyphenyl)guanidine.

16. A compound in accordance with claim 1 which is (3S-trans)-N-(3,4-dichlorophenyl)-N"-cyano-N'-(6-cyano-3,4-dihy- dro-3-hydroxy-2,2-dimethyl-2H-1-benzopyran-4-yl)guanidine.

17. A compound in accordance with claim 1 which is (3S-trans)-N"-cyano-N-(6-cyano-3,4-dihydro-3-hydroxy-2,2-dimethyl-2H-1-benzopyran-4-yl)-N'-[4-(trifluoromethyl)phenyl)]guanidine.

18. The compound in accordance with claim 1 which is (3S-trans)-N"-cyano-N-(6-cyano-3,4-dihydro-3-hydroxy-2,2-dimethyl-2H-1-benzopyran-4-yl)-N'-(4-nitrophenyl)guanidine.

19. The compound in accordance with claim 1 which is (3S-trans)-N-(3-nitrophenyl)-N"-cyano-N'-(6-cyano-3,4-dihydro-3-hydroxy-2,2-dimethyl-2H-1-benzopyran-4-yl)guanidine.

20. The compound in accordance with claim 1 which is (3S-trans)-N-(3-trifluoromethylphenyl)-N"-cyano-N'-(6-cyano-3,4-dihydro-3-hydroxy-2,2-dimethyl-2H-1-benzopyran-4-yl)guanidine.

21. The compound in accordance with claim 1 which is (3S-trans)-N'-cyano-N-(6-cyano-3,4-dihydro-3-hydroxy-2,2-dimethyl-2H-1-benzopyran-4-yl)-N'-(3-methylphenyl)guanidine.

22. The compound in accordance with claim 1 which is (3S-trans)-N"-cyano-N-(6-cyano-3,4-dihydro-3-hydroxy-2,2-dimethyl-2H-1-benzopyran-4-yl)-N'-(4-methylphenyl)guanidine.

23. A process for preparing a compound in accordance with claim 1,
which process comprises coupling a compound of the formula 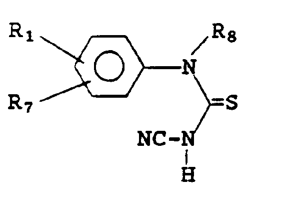 with a compound of the formula 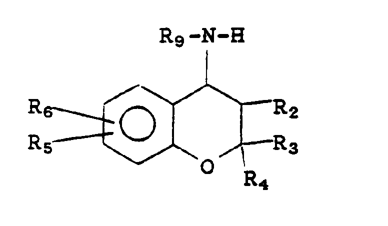 in the presence of a carbodiimide.

24. The process of claim 23 wherein said carbodiimide is of the formula 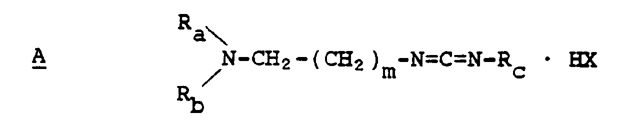 wherein X is halogen, Rₐ, R_{b} and R_{c} are independently alkyl, cycloalkyl, phenyl, phenylalkyl, cycloalkylalkyl or Rₐ and R_{b} together with the N-atom form 1-pyrrolidinyl, 1-piperidinyl, 4-morpholinyl, 4-thiomorpholinyl, 4-alkyl-1-piperazinyl or 4-phenylalkyl-1-piperazinyl.

25. The process of claim 23 wherein said carbodiimide is 1-(3-dimethylaminopropyl)-3-ethylcarbodiimide hydrochloride.

26. The process of claim 23, 24 or 25 wherein the product compound is a compound of any one of claims 2-22.

27. The compound of any one of claims 1-22 for use in the treatment of cardiovascular conditions.

28. Use of the compound of any one of claims 1-22 for the manufacture of a medicament for treating cardiovascular conditions.

29. Use of the compound of any one of claims 1-22 for the manufacture of a medicament for protecting an organ from ischemic damage in a mammalian species subject to an organ surgery procedure.

30. The use of claim 29 wherein said procedure is cardiopulmonary bypass surgery.

31. The use of claim 29 wherein said procedure is organ transplant surgery

32. The use of claim 31 wherein said procedure is heart transplant surgery.

33. The use of claim 29 wherein said compound is added to a solution used in said procedure to preserve, protect or maintain organ function.

34. The use of claim 33 wherein a cardiac-protecting amount of said compound is added to a cardioplegic solution used to arrest, perfuse, store and/or protect a heart involved in a cardiopulmonary bypass or heart transplant procedure.

35. The use of claim 29 wherein said compound is administered to a mammalian specie undergoing an organ surgery procedure before and/or during and/or after said procedure.

36. The use of claims 31 or 32 wherein said compound is administered to an organ donor before and/or during and/or after removal of said organ from said donor.

37. A cardioplegic solution comprising a cardiac-protecting amount of a compound of any one of claims 1-22 and a suitable carrier therefor.

38. A method for protecting a mammalian organ from ischemic damage outside the body, which method comprises employing an organ protecting amount of a compound of any one of claims 1-22.

39. The method of claim 38 wherein the organ is a heart to be used in heart transplant surgery

40. The method of claim 38 or 39 wherein said compound is added to a solution used in said procedure to preserve, protect or maintain organ function.

## Claims (Claims for the following Contracting State(s) : ES, GR)

1. A process for preparing a compound of the formula 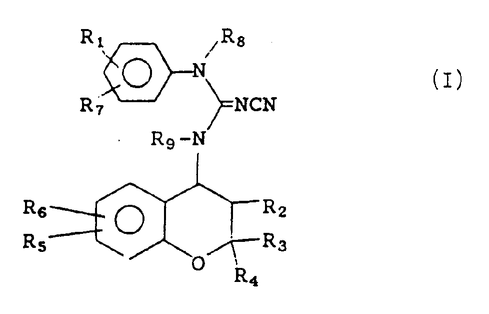
R₁ and R₇ are each independently hydrogen cyano, alkoxy, nitro, halo, hydroxy, haloalkyl, benzyloxy, with the proviso that at least one of R₁ and R₇ is other than hydrogen;
R₂ is hydrogen, hydroxy, 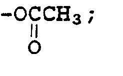
R₃ and R₄ are each independently hydrogen, alkyl or arylalkyl, or, R₃ and R₄ taken together with the carbon atom to which they are attached form a 5- to 7-membered carbocyclic ring;
R₅ is selected from H, alkyl, haloalkyl, alkenyl, alkynyl, cycloalkyl, arylalkyl, cycloalkylalkyl, -CN, -NO_{z}, -COR, -COOR, -CONHR, -CONR₂, -CF₃, S-alkyl, -SOalkyl, -SO₂alkyl, 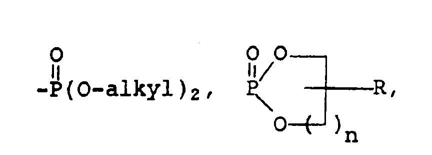 halogen, amino, substituted amino, O-alkyl, OCF₃, OCH₂CF₃, -OCOalkyl, -OCONRalkyl, -NRCOalkyl and NRCOOalkyl, NRCONR₂ wherein R in each of the above groups can be hydrogen, alkyl, haloalkyl, aryl, arylalkyl, cycloalkyl, or (cycloalkyl)alkyl;
R₆ is selected from H, alkyl, OH, O-alkyl, amino, substituted amino, NHCOR (wherein R is as defined above), CN, and NO₂;
R₈ is selected from hydrogen, alkyl;
Rg is selected from hydrogen, alkyl, alkenyl, aryl, arylalkyl, cycloalkyl or cycloalkylalkyl; and
n is 1,2 or 3;
wherein:
"alkyl", "alkenyl", "alkynyl" and "alkoxy" refer to such groups having up to eight carbons;
"cycloalkyl" refers to such groups having three to seven carbons;
"halo" and "halogen" refer to chloro, bromo and fluoro;
"aryl" refers to phenyl, 1-naphthyl, 2-naphthyl; mono substituted phenyl, 1-naphthyl, or 2-naphthyl wherein said substituent is alkyl of 1 to 4 carbons, alkylthio of 1 to 4 carbons, alkoxy of 1 to 4 carbons, halo, nitro, cyano, hydroxy, amino, -NH-alkyl wherein alkyl is of 1 to 4 carbons, -N(alkyl)₂ wherein alkyl is of 1 to 4 carbons, -CF₃, -OCHF₂, 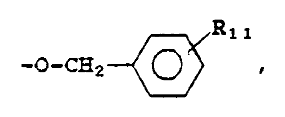 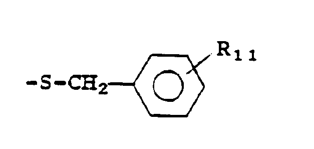 (wherein R₁₁ is hydrogen,
alkyl of 1 to 4 carbons, alkoxy of 1 to 4 carbons, alkylthio of 1 to 4 carbons, halo, hydroxy or CF₃), -O-CH₂-cycloalkyl, or -S-CH₂- cycloalkyl; and di-substituted phenyl, 1-naphthyl, 2-naphthyl wherein said substituents are selected from methyl, methoxy, methylthio, halo, CF₃, nitro, amino, and OCHF₂; and
"substituted amino" refers to a group of the formula -NZ₁Z₂ wherein Z₁ is hydrogen, alkyl, cycloalkyl, aryl, arylalkyl or cycloalkylalkyl, and Z₂ is alkyl, cycloalkyl, aryl, arylalkyl or cycloalkylalkyl, or Z₁ and Z₂ taken together with the nitrogen atom to which they are attached are 1-pyrrolidinyl, 1-piperidinyl, 1-azepinyl, 4-morpholinyl, 4-thiamorpholinyl, 1-piperazinyl, 4-alkyl-1-piperazinyl, 4-arylalkyl-1-piperazinyl, 4-diarylalkyl-1-piperazinyl, or 1-pyrrolidinyl, 1-piperidinyl, or 1-azepinyl substituted with alkyl, alkoxy, alkylthio, halo, trifluoromethyl or hydroxy;
which process comprises coupling a compound of the formula 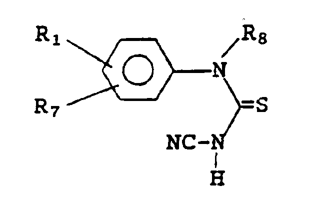 with a compound of the formula 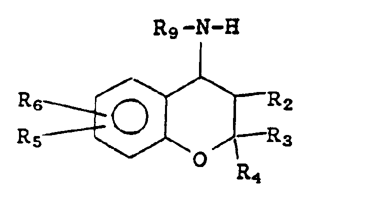 in the presence of a coupling agent.
A process in accordance with claim 1 wherein
R₁ is chloro or fluoro;
R₂ is trans-hydroxy;
R₃ and R₄ are each methyl;
R₅ is -CN or -NO₂;
R₆ is hydrogen;
R₇ is hydrogen;
R₈ is hydrogen; and,
Rg is hydrogen.
A process in accordance with claim 1 wherein the product (I) has the formula 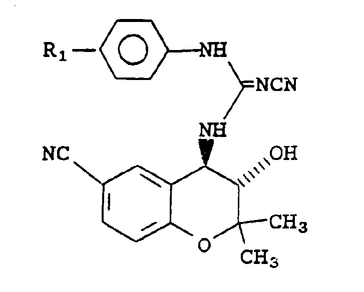
where R₁ is fluoro or chloro.
A process in accordance with claim 1 wherein the product (I) is (trans)-N"-cyano-N-(6-cyano-3,4-dihydro-3-hydroxy-2,2-dimethyl-2H-1-benzopyran-4-yl)-N'-(4-cyanophenyl)guanidine.

5. A process in accordance with claim 1 wherein the product (I) is (frans)-N"-cyano-N-(6-cyano-3,4-dihydro-3-hydroxy-2,2-dimethyl-2H-1-benzopyran-4-yl)-N'-(4-methoxyphenyl)guanidine.

6. A process in accordance with claim 1 wherein the product (I) is (trans)-N°-cyano-N-(6-cyano-3,4-dihydro-3-hydroxy-2,2-dimethyl-2H-1-benzopyran-4-yl)-N'-(4-nitrophenyl)guanidine.

7. A process in accordance with claim 1 wherein the product (I) is (trans)-N-(4-chlorophenyl)-N"-cyano-N-(6-cyano-3,4-dihydro-3-hydroxy-2,2-dimethyl-2H-1-benzopyran-4-yl)guanidine.

8. A process in accordance with claim 1 wherein the product (I) is (trans)-N-(2-chlorophenyl)-N"-cyano-N-(6-cyano-3,4-dihydro-3-hydroxy-2,2-dimethyl-2H-1-benzopyran-4-yl)guanidine.

9. A process in accordance with claim 1 wherein the product (I) is (trans)-N-(3-chlorophenyl)-N"-cyano-N-(6-cyano-3,4-dihydro-3-hydroxy-2,2-dimethyl-2H-1-benzopyran-4-yl)-guanidine.

10. A process in accordance with claim 1 wherein the product (I) is (trans)-N-(4-fluorophenyl)-N"-cyano-N-(6-cyano-3,4-dihydro-3-hydroxy-2,2-dimethyl-2H-1-benzopyran-4-yl)guanidine.

11. A process in accordance with claim 1 wherein the product (I) is (3S-trans)-N"-cyano-N-(6-cyano-3,4-dihydro-3-hydroxy-2,2-dimethyl-2H-1-benzopyran-4-yl)-N'-(4-fluorophenyl)guanidine.

12. A process in accordance with claim 1 wherein the product (I) is (3S-trans)-N-(4-chlorophenyl)-N"-cyano-N'-(6-cyano-3,4-dihydro-3-hydroxy-2,2-dimethyl-2H-1-benzopyran-4-yl)guanidine.

13. A process in accordance with claim 1 wherein the product (I) is (3S-trans)-N-(3-chlorophenyl)-N"-cyano-N'-(6-cyano-3,4-dihydro-3-hydroxy-2,2-dimethyl-2H-1-benzopyran-4-yl)guanidine.

14. A process in accordance with claim 1 wherein the product (I) is trans-N"-cyano-N-(6-cyano-3,4-dihydro-3-hydroxy-2,2-dimethyl-2H-1-benzopyran-4-yl)-N'-[4-(phenylmethoxy)phenyl)guanidine.

15. A process in accordance with claim 1 wherein the product (I) is frans-N"-cyano-N-(6-cyano-3,4-dihydro-3-hydroxy-2,2-dimethyl-2H-1-benzopyran-4-yl)-N'-(4-hydroxyphenyl)guanidine.

16. A process in accordance with claim 1 wherein the product (I) is (3S-trans)-N-(3,4-dichlorophenyl)-N"-cyano-N'-(6-cyano-3,4-dihydro-3-hydroxy-2,2-dimethyl-2H-1-benzopyran-4-yl)guanidine.

17. A process in accordance with claim 1 wherein the product (I) is (3S-trans)-N"-cyano-N-(6-cyano-3,4tiihydro-3-hydroxy-2,2-dimethyl-2H-1-benzopyran-4-yl)-N'-[4-(trifluoromethyl)phenyl)]guanidine.

18. A process in accordance with claim 1 wherein the product (I) is (3S-trans)-N"-cyano-N-(6-cyano-3,4-dihydro-3-hydroxy-2,2-dimethyl-2H-1-benzopyran-4-yl)-N'-(4-nitrophenyl)guanidine.

19. A process in accordance with claim 1 wherein the product (I) is (3S-trans)-N-(3-nitrophenyl)-N"-cyano-N'-(6-cyano-3,4-dihydro-3-hydroxy-2,2-dimethyl-2H-1-benzopyran-4-yl)guanidine.

20. A process in accordance with claim 1 wherein the product (I) is (3S-trans)-N-(3-trifluoromethylphenyl)-N"-cyano-N'-(6-cyano-3,4-dihydro-3-hydroxy-2,2-dimethyl-2H-1-benzopyran-4-yl)guanidine.

21. A process in accordance with claim 1 wherein the product (I) is (3S-trans)-N'-cyano-N-(6-cyano-3,4₋dihydro-3-hydroxy-2,2-dimethyl-2H-1-benzopyran-4-yl)-N'-(3-methylphenyl)guanidine.

22. A process in accordance with claim 1 wherein the product (I) is (3S-trans)-N_{"}-cyano-N-(6-cyano-3,4-dihydro-3-hydroxy-2,2-dimethyl-2H-1-benzopyran-4-yl)-N'-(4-methylphenyl)guanidine.

23. The process of any preceding claim wherein the coupling agent is a carbodiimide.

24. The process of claim 23 wherein said carbodiimide is of the formula 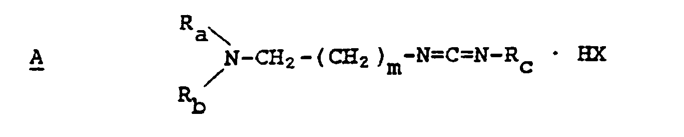 wherein X is halogen, Rₐ, R_{b} and R_{c} are independently alkyl, cycloalkyl, phenyl, phenylalkyl, cycloalkylalkyl or Rₐ and R_{b} together with the N-atom form 1-pyrrolidinyl, 1-piperidinyl, 4-morpholinyl, 4-thiomorpholinyl, 4-alkyl-1-piperazinyl or 4-phenylalkyl-1-piperazinyl.

25. The process of claim 23 wherein said carbodiimide is 1-(3-dimethylaminopropyl)-3-ethyl-carbodiimide hydrochloride.

26. A method for protecting a mammalian organ from ischemic damage outside the body, which method comprises employing an organ protecting amount of a compound of formula I as defined in any one of claims 1-22.

27. The method of claim 26 wherein the organ is a heart to be used in heart transplant surgery.

28. The method of claim 26 or 27 wherein said compound is added to a solution used in said procedure to preserve, protect or maintain organ function.

## Patentansprüche (Patentansprüche für folgende(n) Vertragsstaat(en) : AT, BE, CH, LI, DE, DK, FR, GB, IT, LU, MC, NL, PT, SE)

1. Verbindung der Formel 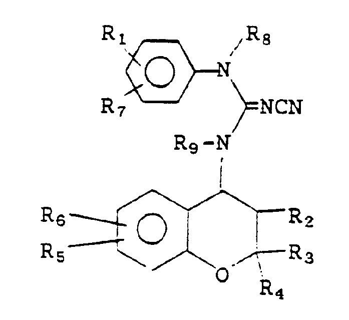
R₁ und R₇ sind jeweils unabhängig voneinander ein Wasserstoffatom, eine Cyano-, Alkoxy-, Nitrogruppe, ein Halogenatom, eine Hydroxyl-, Halogenalkyl- oder Benzyloxygruppe, mit der Maßgabe, daß mindestens einer der Reste R₁ und R₇ kein Wasserstoffatom ist;
R₂ ist ein Wasserstoffatom, eine Hydroxylgruppe oder 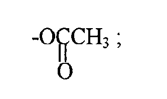
R₃ und R₄ sind jeweils unabhängig voneinander ein Wasserstoffatom, ein Alkyl- oder Arylalkylrest, oder R₃ und R₄ bilden zusammen mit dem Kohlenstoffatom, an das sie gebunden sind, einen 5- bis 7-gliedrigen carbocyclischen Ring;
R₅ ist ausgewählt aus den Resten H, Alkyl-, Halogenalkyl-, Alkenyl-, Alkinyl-, Cycloalkyl-, Arylalkyl-, (Cycloalkyl)alkyl-, -CN, -N0₂, -COR, -COOR, -CONHR, -CONR₂, -CF₃, S-alkyl, -SOalkyl, -SO₂alkyl, 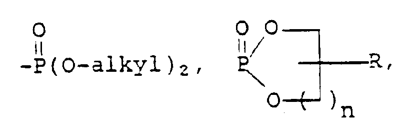 Halogen, Amino-, substituiertes Amino-, -O-alkyl, -OCF₃, -OCH₂CF₃, -OCOalkyl, -OCONRalkyl, -NRCOalkyl, - NRCOOalkyl und -NRCONR₂, wobei R in jedem der vorstehenden Reste ein Wasserstoffatom, ein Alkyl-, Halogenalkyl-, Aryl-, Arylalkyl-, Cycloalkyl- oder (Cycloalkyl)alkylrest sein kann;
R₆ ist ausgewählt aus den Resten H, Alkyl-, -OH, -O-alkyl, Amino-, substituiertes Amino-, -NHCOR (wobei R wie vorstehend definiert ist), -CN und -NO_{z};
R₈ ist ausgewählt aus einem Wasserstoffatom und einem Alkylrest;
Rg ist ausgewählt aus einem Wasserstoffatom, einem Alkyl-, Alkenyl-, Aryl-, Arylalkyl-, Cycloalkyl- und (Cycloalkyl)alkylrest; und
n ist 1, 2 oder 3;
wobei:
"Alkyl-", "Alkenyl-", "Alkinyl-" und "Alkoxy-" sich auf derartige Reste mit bis zu 8 Kohlenstoffatomen beziehen;
"Cycloalkyl-" derartige Reste mit 3 bis 7 Kohlenstoffatomen betrifft;
"Halogen-" sich auf Chlor-, Brom- und Fluoratome bezieht;
"Aryl-" sich auf einen Phenyl-, 1-Naphthyl- oder 2-Naphthylrest; einen monosubstituierten Phenyl-, 1-Naphthyl- oder 2-Naphthylrest, wobei der Substituent ein Alkylrest mit 1 bis 4 Kohlenstoffatomen, ein Alkylthiorest mit 1 bis 4 Kohlenstoffatomen, ein Alkoxyrest mit 1 bis 4 Kohlenstoffatomen, ein Halogenatom, eine Nitro-, Cyano-, Hydroxyl-, Aminogruppe, ein -NH-alkylrest, in dem 'alkyl' aus 1 bis 4 Kohlenstoffatomen besteht, ein -N(alkyl)₂-Rest, in dem 'alkyl' aus 1 bis 4 Kohlenstoffatomen besteht, -CF₃, -OCHF₂, 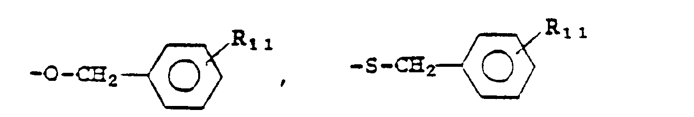 (wobei R₁₁ ein Wasserstoffatom, ein Alkylrest mit 1 bis 4 Kohlenstoffatomen, ein Alkoxyrest mit 1 bis 4 Kohlenstoffatomen, ein Alkylthiorest mit 1 bis 4 Kohlenstoffatomen, ein Halogenatom, eine Hydroxyl- oder CF₃-Gruppe ist), ein Rest -O-CH₂-cycloalkyl oder -S-CH₂-cycloalkyl ist; sowie auf einen disubstituierten Phenyl-, 1-Naphthyl- oder 2-Naphthylrest, wobei die Substituenten aus einer Methyl-, Methoxy-, Methylthiogruppe, einem Halogenatom, einer CF₃-, Nitro-, Amino- und OCHF₂-Gruppe ausgewählt sind, bezieht; und
"substituiertes Amino-" einen Rest der Formel-NZ₁Z₂ betrifft, wobei Z₁ ein Wasserstoffatom, ein Alkyl-, Cycloalkyl-, Aryl-, Arylalkyl- oder (Cycloalkyl)alkylrest ist und Z₂ ein Alkyl-, Cycloalkyl-, Aryl-, Arylalkyl oder (Cycloalkyl)alkylrest ist, oder Z₁ und Z₂ zusammen mit dem Stickstoffatom, an das sie gebunden sind, einen 1-Pyrrolidinyl- , 1-Piperidinyl-, 1-Azepinyl-, 4-Morpholinyl-, 4-Thiomorpholinyl-, 1-Piperazinyl-, 4-Alkyl-1-piperazinyl-, 4-Arylalkyl-1-piperazinyl- oder 4-Diarylalkyl-1-piperazinylrest oder eine 1-Pyrrolidinyl-, 1-Piperidinyl- oder 1-Azepinylgruppe, die mit einem Alkyl-, Alkoxy-, Alkylthiorest, einem Halogenatom, einer Trifluormethyl- oder Hydroxylgruppe substituiert ist, darstellen.

2. Verbindung nach Anspruch 1, wobei
R₁ ein Chlor- oder ein Fluoratom ist;
R₂ eine trans-Hydroxylgruppe ist;
R₃ und R₄ jeweils eine Methylgruppe sind;
R₅ eine CN- oder NO₂-Gruppe ist;
R₆ ein Wasserstoffatom ist;
R₇ ein Wasserstoffatom ist;
R₈ ein Wasserstoffatom ist; und
Rg ein Wasserstoffatom ist.

3. Verbindung nach Anspruch 1 mit der Formel 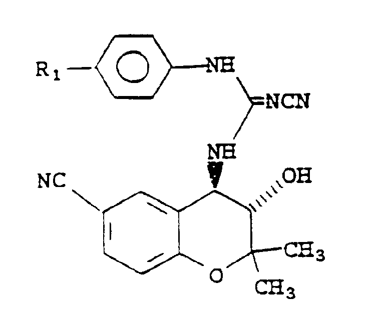 wobei R₁ ein Fluor- oder ein Chloratom ist.

4. Verbindung nach Anspruch 1, bei welcher es sich um (trans)-N"-Cyano-N-(6-cyano-3,4-dihydro-3-hydroxy-2,2-dimethyl-2H-1-benzopyran-4-yl)-N'-(4-cyanophenyl)guanidin handelt.

5. Verbindung nach Anspruch 1, bei welcher es sich um (trans)-N"-Cyano-N-(6-cyano-3,4-dihydro-3-hydroxy-2,2-dimethyl-2H-1-benzopyran-4-yl)-N'-(4-methoxyphenyl)guanidin handelt.

6. Verbindung nach Anspruch 1, bei welcher es sich um (trans)-N"-Cyano-N-(6-cyano-3,4-dihydro-3-hydroxy-2,2-dimethyl-2H-1-benzopyran-4-yl)-N'-(4-nitrophenyl)guanidin handelt.

7. Verbindung nach Anspruch 1, bei welcher es sich um (trans)-N-(4-Chlorphenyl)-N"-cyano-N-(6-cyano-3,4-dihydro-3-hydroxy-2,2-dimethyl-2H-1-benzopyran-4-yl)guanidin handelt.

8. Verbindung nach Anspruch 1, bei welcher es sich um (trans)-N-(2-Chlorphenyl)-N"-cyano-N-(6-cyano-3,4-dihydro-3-hydroxy-2,2-dimethyl-2H-1-benzopyran-4-yl)guanidin handelt.

9. Verbindung nach Anspruch 1, bei welcher es sich um (trans)-N-(3-Chlorphenyl)-N"-cyano-N-(6-cyano-3,4-dihydro-3-hydroxy-2,2-dimethyl-2H-1-benzopyran-4-yl)guanidin handelt.

10. Verbindung nach Anspruch 1, bei welcher es sich um (trans)-N-(4-Fluorphenyl)-N"-cyano-N-(6-cyano-3,4-dihydro-3-hydroxy-2,2-dimethyl-2H-1-benzopyran-4-yl)guanidin handelt.

11. Verbindung nach Anspruch 1, bei welcher es sich um (3S-trans)-N"-Cyano-N-(6-cyano-3,4-dihydro-3-hydroxy-2,2-dimethyl-2H-1-benzopyran-4-yl)-N'-(4-fluorphenyl)guanidin handelt.

12. Verbindung nach Anspruch 1, bei welcher es sich um (3S-trans)-N-(4-Chlorphenyl)-N"-cyano-N'-(6-cyano-3,4-dihy- dro-3-hydroxy-2,2-dimethyl-2H-1-benzopyran-4-yl)guanidin handelt.

13. Verbindung nach Anspruch 1, bei welcher es sich um (3S-trans)-N-(3-Chlorphenyl)-N"-cyano-N'-(6-cyano-3,4-dihy- dro-3-hydroxy-2,2-dimethyl-2H-1-benzopyran-4-yl)guanidin handelt.

14. Verbindung nach Anspruch 1, bei welcher es sich um (trans)-N"-Cyano-N-(6-cyano-3,4-dihydro-3-hydroxy-2,2-dimethyl-2H-1-benzopyran-4-yl)-N'-[4-(phenylmethoxy)-phenyllguanidin handelt.

15. Verbindung nach Anspruch 1, bei welcher es sich um (trans)-N"-Cyano-N-(6-cyano-3,4-dihydro-3-hydroxy-2,2-dimethyl-2H-1-benzopyran-4-yl)-N'-(4-hydroxyphenyl)guanidin handelt.

16. Verbindung nach Anspruch 1, bei welcher es sich um (3S-trans)-N-(3,4-Dichlorphenyl)-N"-cyano-N'-(6-cyano-3,4-dihydro-3-hydroxy-2,2-dimethyl-2H-1-benzopyran-4-yl)guanidin handelt.

17. Verbindung nach Anspruch 1, bei welcher es sich um (3S-trans)-N"-Cyano-N-(6-cyano-3,4-dihydro-3-hydroxy-2,2-dimethyl-2H-1-benzopyran-4-yl)-N'-[4-(trifluormethyl)-phenyl]guanidin handelt.

18. Verbindung nach Anspruch 1, bei welcher es sich um (3S-trans)-N"-Cyano-N-(6-cyano-3,4-dihydro-3-hydroxy-2,2-dimethyl-2H-1-benzopyran-4-yl)-N'-(4-nitrophenyl)guanidin handelt.

19. Verbindung nach Anspruch 1, bei welcher es sich um (3S-trans)-N-(3-Nitrophenyl)-N"-cyano-N'-(6-cyano-3,4-dihy- dro-3-hydroxy-2,2-dimethyl-2H-1-benzopyran-4-yl)guanidin handelt.

20. Verbindung nach Anspruch 1, bei welcher es sich um (3S-trans)-N-(3-Trifluormethylphenyl)-N"-cyano-N'-(6-cyano-3,4-dihydro-3-hydroxy-2,2-dimethyl-2H-1-benzopyran-4-yl)guanidin handelt.

21. Verbindung nach Anspruch 1, bei welcher es sich um (3S-trans)-N'-Cyano-N-(6-cyano-3,4-dihydro-3-hydroxy-2,2-dimethyl-2H-1-benzopyran-4-yl)-N'-(3-methylphenyl)guanidin handelt.

22. Verbindung nach Anspruch 1, bei welcher es sich um (3S-trans)-N"-Cyano-N-(6-cyano-3,4-dihydro-3-hydroxy-2,2-dimethyl-2H-1-benzopyran-4-yl)-N'-(4-methylphenyl)guanidin handelt.

23. Verfahren zur Herstellung einer Verbindung nach Anspruch 1, wobei das Verfahren die Verknüpfung einer Verbindung der Formel 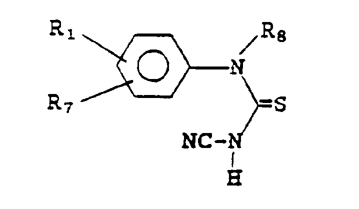 mit einer Verbindung der Formel 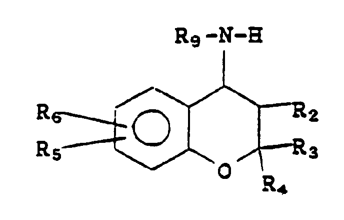 in Gegenwart von Carbodiimid umfaßt.

24. Verfahren aus Anspruch 23, wobei das Carbodiimid die Formel 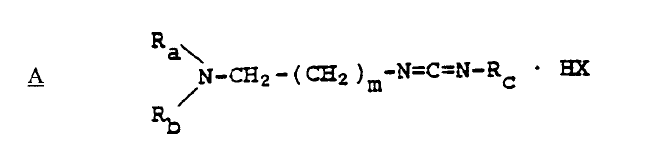 hat, worin X ein Halogenatom ist, Rₐ, R_{b} und Rₑ unabhängig voneinander Alkyl-, Cycloalkyl-, Phenyl-, Phenylalkyl-oder (Cycloalkyl)alkylreste sind, oder Rₐ und R_{b} zusammen mit dem N-Atom eine 1-Pyrrolidinyl-, 1-Piperidinyl-, 4-Morpholinyl-, 4-Thiomorpholinyl-, 4-Alkyl-1-piperazinyl- oder 4-Phenylalkyl-1-piperazinyl-Einheit bilden.

25. Verfahren nach Anspruch 23, wobei das Carbodiimid 1-(3-Dimethylaminopropyl)-3-ethylcarbodiimid-Hydrochlorid ist.

26. Verfahren nach Anspruch 23, 24 oder 25, wobei die Produktverbindung eine Verbindung nach einem der Ansprüche 2 - 22 ist.

27. Verbindung nach einem der Ansprüche 1 - 22 zur Verwendung bei der Behandlung kardiovaskulärer Zustände.

28. Verwendung der Verbindung nach einem der Ansprüche 1 - 22 zur Herstellung eines Medikamentes für die Behandlung kardiovaskulärer Zustände.

29. Verwendung der Verbindung nach einem der Ansprüche 1 - 22 für die Herstellung eines Medikamentes zum Schutz eines Organs in einer Säugetierspezies, die einem organchirurgischen Eingriff unterliegt, vor ischämischen Schäden.

30. Verwendung nach Anspruch 29, wobei der Eingriff eine kardiopulmonale Bypass-Operation ist.

31. Verwendung nach Anspruch 29, wobei der Eingriff eine Organtransplantation ist.

32. Verwendung nach Anspruch 31, wobei der Eingriff eine Herztransplantation ist.

33. Verwendung nach Anspruch 29, wobei die Verbindung einer Lösung zugesetzt wird, welche bei dem Eingriff zum Konservieren, Schützen oder Aufrechterhalten der Organfunktion verwendet wird.

34. Verwendung nach Anspruch 33, wobei eine herzschützende Menge der Verbindung einer kardioplegischen Lösung zugesetzt wird, die verwendet wird, um ein Herz im Verlauf einer kardiopulmonalen Bypass-Operation oder einer Herztransplantation zum Stillstand zu bringen, zu durchströmen, aufzubewahren und/oder zu schützen.

35. Verwendung nach Anspruch 29, wobei die Verbindung einer Säugetierspezies, welche einem organchirurgischen Eingriff unterliegt, vor und/oder während und/oder nach dem Eingriffverabreicht wird.

36. Verwendung nach den Ansprüchen 31 oder 32, wobei die Verbindung einem Organspender vor und/oder während und/oder nach der Entfernung des Organs vom Organspender verabreicht wird.

37. Eine kardioplegische Lösung, umfassend eine herzschützende Menge einer Verbindung nach einem der Ansprüche 1 - 22 und einen geeigneten Träger dafür.

38. Verfahren zum Schutz eines Säugetierorgans vor ischämischen Schäden außerhalb des Körpers, wobei das Verfahren den Einsatz einer organschützenden Menge einer Verbindung nach einem der Ansprüche 1 - 22 umfaßt.

39. Verfahren nach Anspruch 38, wobei das Organ ein zur Verwendung in einer Herztransplantation vorgesehenes Herz ist.

40. Verfahren nach Anspruch 38 oder 39, wobei die Verbindung einer Lösung zugesetzt wird, welche bei dem Eingriff zum Konservieren, Schützen oder Aufrechterhalten der Organfunktion verwendet wird.

## Patentansprüche (Patentansprüche für folgende(n) Vertragsstaat(en) : ES, GR)

1. Verfahren zur Herstellung einer Verbindung der Formel 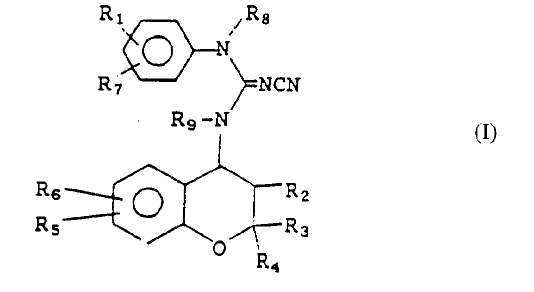
R₁ und R₇ sind jeweils unabhängig voneinander ein Wasserstoffatom, eine Cyano-, Alkoxy-, Nitrogruppe, ein Halogenatom, eine Hydroxyl-, Halogenalkyl- oder Benzyloxygruppe, mit der Maßgabe, daß mindestens einer der Reste R₁ und R₇ kein Wasserstoffatom ist;
R₂ ist ein Wasserstoffatom, eine Hydroxylgruppe oder 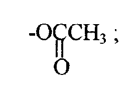
R₃ und R₄ sind jeweils unabhängig voneinander ein Wasserstoffatom, ein Alkyl- oder Arylalkylrest, oder R₃ und R₄ bilden zusammen mit dem Kohlenstoffatom, an das sie gebunden sind, einen 5- bis 7-gliedrigen carbocyclischen Ring;
R₅ ist ausgewählt aus den Resten H, Alkyl-, Halogenalkyl-, Alkenyl-, Alkinyl-, Cycloalkyl-, Arylalkyl-, (Cycloalkyl)alkyl-, -CN, -NO₂, -COR, -COOR, -CONHR, -CONR₂, -CF₃, -S-alkyl, -SOalkyl, -SO₂alkyl, 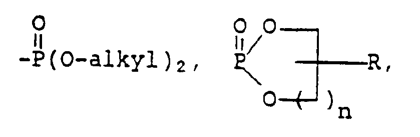 Halogen, Amino-, substituiertes Amino-, -O-alkyl, -OCF₃, -OCH₂CF₃, -OCOalkyl, -OCONRalkyl, -NRCOalkyl, - NRCOOalkyl und -NRCONR₂, wobei R in jedem der vorstehenden Reste ein Wasserstoffatom, ein Alkyl-, Halogenalkyl-, Aryl-, Arylalkyl-, Cycloalkyl- oder (Cycloalkyl)alkylrest sein kann;
R₆ ist ausgewählt aus den Resten H, Alkyl-, -OH, -O-alkyl, Amino-, substituiertes Amino-, -NHCOR (wobei R wie vorstehend definiert ist), -CN und -NO₂;
R₈ ist ausgewählt aus einem Wasserstoffatom und einem Alkylrest;
Rg ist ausgewählt aus einem Wasserstoffatom, einem Alkyl-, Alkenyl-, Aryl-, Arylalkyl-, Cycloalkyl- oder (Cycloalkyl)alkylrest; und
n ist 1, 2 oder 3;
wobei :
"Alkyl-", "Alkenyl-", "Alkinyl-" und "Alkcxy." sich auf derartige Reste mit bis zu 8 Kohlenstoffatomen beziehen;
"Cycloalkyl-" derartige Reste mit 3 bis 7 Kohlenstoffatomen betrifft;
"Halogen-" sich auf Chlor-, Brom- und Fluoratome bezieht;
"Aryl-" sich auf einen Phenyl-, 1-Naphthyl- oder 2-Naphthylrest; einen monosubstituierten Phenyl-, 1-Naphthyl- oder 2-Naphthylrest, wobei der Substituent ein Alkylrest mit 1 bis 4 Kohlenstoffatomen, ein Alkylthiorest mit 1 bis 4 Kohlenstoffatomen, ein Alkoxyrest mit 1 bis 4 Kohlenstoffatomen, ein Halogenatom, eine Nitro-, Cyano-, Hydroxyl-, Aminogruppe, ein -NH-alkylrest, in dem 'alkyl' aus 1 bis 4 Kohlenstoffatomen besteht, ein -N(alkyl)₂-Rest, in dem 'alkyl' aus 1 bis 4 Kohlenstoffatomen besteht, -CF₃, -OCHF₂, 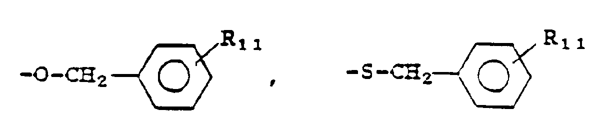 (wobei R₁₁ ein Wasserstoffatom, ein Alkylrest mit 1 bis 4 Kohlenstoffatomen, ein Alkoxyrest mit 1 bis 4 Kohlenstoffatomen, ein Alkylthiorest mit 1 bis 4 Kohlenstoffatomen, ein Halogenatom, eine Hydroxyl- oder CF₃-Gruppe ist), ein Rest -O-CH₂-cycloalkyl oder -S-CH₂-cycloalkyl ist; sowie auf einen disubstituierten Phenyl-, 1-Naphthyl- oder 2-Naphthylrest, wobei die Substituenten aus einer Methyl-, Methoxy-, Methylthiogruppe, einem Halogenatom, einer CF₃-, Nitro-, Amino- und OCHF₂-Gruppe ausgewählt sind, bezieht; und
substituiertes Amino-" einen Rest der Formel -NZ₁Z₂ betrifft, wobei Z₁ ein Wasserstoffatom, ein Alkyl-, Cycloalkyl-, Aryl-, Arylalkyl- oder (Cycloalkyl)alkylrest ist und Z₂ ein Alkyl-, Cycloalkyl-, Aryl-, Arylalkyl oder (Cycloalkyl)alkylrest ist, oder Z₁ und Z₂ zusammen mit dem Stickstoffatom, an das sie gebunden sind, einen 1-Pyrrolidinyl- , 1-Piperidinyl-, 1-Azepinyl-, 4-Morpholinyl-, 4-Thiomorpholinyl-, 1-Piperazinyl-, 4-Alkyl-1-piperazinyl-, 4-Arylalkyl-1-piperazinyl- oder 4-Diarylalkyl-1-piperazinylrest oder eine 1-Pyrrolidinyl-, 1-Piperidinyl- oder 1-Azepinylgruppe, die mit einem Alkyl-, Alkoxy-, Alkylthiorest, einem Halogenatom, einer Trifluormethyl- oder Hydroxylgruppe substituiert ist, darstellen;
wobei das Verfahren das Verknüpfen einer Verbindung der Formel 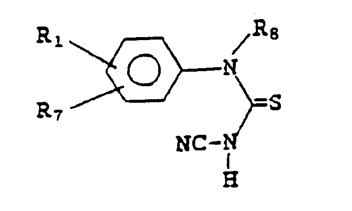 mit einer Verbindung der Formel 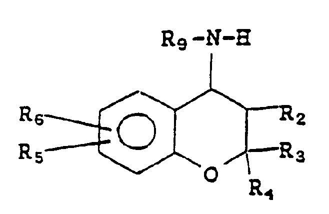 in Gegenwart eines Kupplungsmittels umfaßt.

2. Verfahren nach Anspruch 1, wobei
R₁ ein Chlor- oder ein Fluoratom ist;
R₂ eine trans-Hydroxylgruppe ist;
R₃ und R₄ jeweils eine Methylgruppe sind;
R₅ eine CN- oder NO₂-Gruppe ist;
R₆ ein Wasserstoffatom ist;
R₇ ein Wasserstoffatom ist;
R₈ ein Wasserstoffatom ist; und
Rg ein Wasserstoffatom ist.

3. Verfahren nach Anspruch 1, wobei das Produkt (I) die Formel 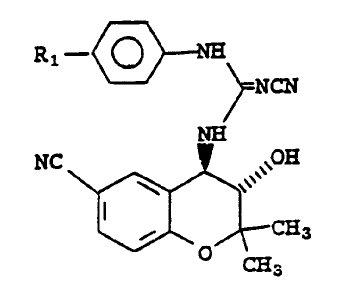 hat, in der R₁ ein Fluor- oder ein Chloratom ist.

4. Verfahren nach Anspruch 1, wobei das Produkt (I) (trans)-N"-Cyano-N-(6-cyano-3,4-dihydro-3-hydroxy-2,2-dimethyl-2H-1-benzopyran-4-yl)-N'-(4-cyanophenyl)guanidin ist.

5. Verfahren nach Anspruch 1, wobei das Produkt (I) (trans)-N"-Cyano-N-(6-cyano-3,4-dihydro-3-hydroxy-2,2-dimethyl-2H-1-benzopyran-4-yl)-N'-(4-methoxyphenyl)guanidin ist.

6. Verfahren nach Anspruch 1, wobei das Produkt (I) (trans)-N"-Cyano-N-(6-cyano-3,4-dihydro-3-hydroxy-2,2-dimethyl-2H-1 -benzopyran-4-yl)-N'-(4-nitrophenyl)guanidin ist.

7. Verfahren nach Anspruch 1, wobei das Produkt (I) (trans)-N-(4-Chlorphenyl)-N"-cyano-N-(6-cyano-3,4-dihydro-3-hydroxy-2,2-dimethyl-2H-1-benzopyran-4-yl)guanidin ist.

8. Verfahren nach Anspruch 1, wobei das Produkt (I) (trans)-N-(2-Chlorphenyl)-N"-cyano-N-(6-cyano-3,4-dihydro-3-hydroxy-2,2-dimethyl-2H-1-benzopyran-4-yl)guanidin ist.

9. Verfahren nach Anspruch 1, wobei das Produkt (I) (trans)-N-(3-Chlorphenyl)-N"-cyano-N-(6-cyano-3,4-dihydro-3-hydroxy-2,2-dimethyl-2H-1-benzopyran-4-yl)guanidin ist.

10. Verfahren nach Anspruch 1, wobei das Produkt (I) (trans)-N-(4-Fluorphenyl)-N"-cyano-N-(6-cyano-3,4-dihydro-3-hydroxy-2,2-dimethyl-2H-1-benzopyran-4-yl)guanidin ist.

11. Verfahren nach Anspruch 1, wobei das Produkt (I) (3S-trans)-N"-Cyano-N-(6-cyano-3,4-dihydro-3-hydroxy-2,2-dimethyl-2H-1-benzopyran-4-yl)-N'-(4-fiuorphenyl)guanidin ist.

12. Verfahren nach Anspruch 1, wobei das Produkt (I) (3S-trans)-N-(4-Chlorphenyl)-N"-cyano-N'-(6-cyano-3,4-dihydro-3-hydroxy-2,2-dimethyl-2H-1-benzopyran-4-yl)guanidin ist.

13. Verfahren nach Anspruch 1, wobei das Produkt (I) (3S-trans)-N-(3-Chlorphenyl)-N"-cyano-N'-(6-cyano-3,4-dihydro-3-hydroxy-2,2-dimethyl-2H-1-benzopyran-4-yl)guanidin ist.

14. Verfahren nach Anspruch 1, wobei das Produkt (I) (trans)-N"-Cyano-N-(6-cyano-3,4-dihydro-3-hydroxy-2,2-dimethyl-2H-1-benzopyran-4-yl)-N'-[4-(phenylmethoxy)phenyl]guanidin ist.

15. Verfahren nach Anspruch 1, wobei das Produkt (I) (trans)-N"-Cyano-N-(6-cyano-3,4-dihydro-3-hydroxy-2,2-dimethyl-2H-1 -benzopyran-4-yl)-N'-(4-hydroxyphenyl)guanidin ist.

16. Verfahren nach Anspruch 1, wobei das Produkt (I) (3S-trans)-N-(3,4-Dichlorphenyl)-N"-cyano-N'-(6-cyano-3,4-dihy- dro-3-hydroxy-2,2-dimethyl-2H-1 -benzopyran-4-yl)guanidin ist.

17. Verfahren nach Anspruch 1, wobei das Produkt (I) (3S-trans)-N"-Cyano-N-(6-cyano-3,4-dihydro-3-hydroxy-2,2-dimethyl-2H-1-benzopyran-4-yl)-N'-[4-(trifluormethyl)phenyl]guanidin ist.

18. Verfahren nach Anspruch 1, wobei das Produkt (I) (3S-trans)-N"-Cyano-N-(6-cyano-3,4-dihydro-3-hydroxy-2,2-dimethyl-2H-1-benzopyran-4-yl)-N'-(4-nitrophenyl)guanidin ist.

19. Verfahren nach Anspruch 1, wobei das Produkt (I) (3S-trans)-N-(3-Nitrophenyl)-N"-cyano-N'-(6-cyano-3,4-dihydro-3-hydroxy-2,2-dimethyl-2H-1-benzopyran-4-yl)guanidin ist.

20. Verfahren nach Anspruch 1, wobei das Produkt (I) (3S-trans)-N-(3-Trirfluormethylphenyl)-N"-cyano-N'-(6-cyano-3,4-dihydro-3-hydroxy-2,2-dimethyl-2H-1-benzopyran-4-yl)guanidin ist.

21. Verfahren nach Anspruch 1, wobei das Produkt (I) (3S-trans)-N'-Cyano-N-(6-cyano-3,4-dihydro-3-hydroxy-2,2-dimethyl-2H-1-benzopyran-4-yl)-N'-(3-methylphenyl)guanidin ist.

22. Verfahren nach Anspruch 1, wobei das Produkt (I) (3S-trans)-N"-Cyano-N-(6-cyano-3,4-dihydro-3-hydroxy-2,2-dimethyl-2H-1-benzopyran-4-yl)-N'-(4-methylphenyl)guanidin ist.

23. Verfahren nach einem der vorausgehenden Ansprüche, wobei das Kupplungsmittel ein Carbodiimid ist.

24. Verfahren aus Anspruch 23, wobei das Carbodiimid die Formel 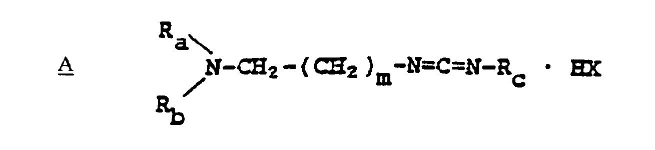 hat, worin X ein Halogenatom ist, Rₐ, R_{b} und R_{c} unabhängig voneinander Alkyl-, Cycloalkyl-, Phenyl-, Phenylalkyl-oder (Cycloalkyl)alkylreste sind, oder Rₐ und R_{b} zusammen mit dem N-Atom eine 1-Pyrrolidinyl-, 1-Piperidinyl-, 4-Morpholinyl, 4-Thiomorpholinyl-, 4-Alkyl-1-piperazinyl- oder 4-Phenylalkyl-1-piperazinyl-Einheit bilden.

25. Verfahren nach Anspruch 23, wobei das Carbodiimid 1-(3-Dimethylaminopropyl)-3-ethylcarbodiimid-Hydrochlorid ist.

26. Verfahren zum Schutz eines Säugetierorgans vor ischämischen Schäden außerhalb des Körpers, wobei das Verfahren den Einsatz einer organschützenden Menge einer Verbindung der Formel (I), wie in einem der Ansprüche 1 - 22 definiert, umfaßt.

27. Verfahren nach Anspruch 26, wobei das Organ ein zur Verwendung in einer Herztransplantation vorgesehenes Herz ist.

28. Verfahren nach Anspruch 26 oder 27, wobei die Verbindung einer Lösung zugesetzt wird, welche bei dem Eingriff zum Konservieren, Schützen oder Aufrechterhalten der Organfünktion verwendet wird.

## Revendications (Revendications pour l'(les) Etat(s) contractant(s) suivant(s) : AT, BE, CH, LI, DE, DK, FR, GB, IT, LU, MC, NL, PT, SE)

1. Composé de formule 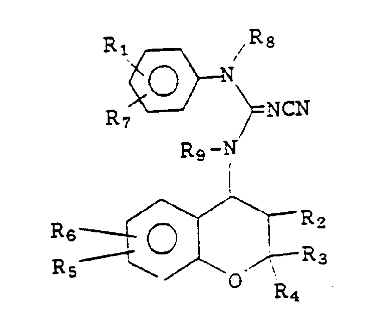
R₁ et R₇ sont chacun, indépendamment, l'hydrogène ou un substituant cyano, alcoxy, nitro, halo, hydroxy, haloalkyle ou benzyloxy, sous réserve qu'au moins l'un de R₁ et R₇ soit autre que l'hydrogène;
R₂ est l'hydrogène ou un groupe hydroxy ou 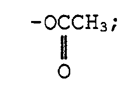
R₃ et R₄ sont chacun, indépendamment, l'hydrogène ou un groupe alkyle ou arylalkyle, ou bien R₃ et R₄ forment, avec l'atome de carbone auquel ils sont fixés, un noyau carbocyclique de 5 à 7 chaînons;
R₅ est choisi entre l'hydrogène et les substituants alkyle; haloalkyle, alcényle, alcynyle, cycloalkyle, arylalkyle, cycloalkylalkyle, -CN, -N0₂, -COR, -COOR, -CONHR, -CONR₂, -CF₃, S-alkyle, -SOalkyle, -SO₂alkyle, 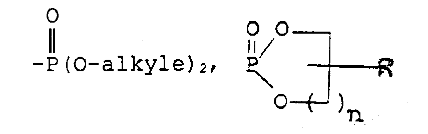 halogène, amine, amine substitué, O-alkyle, OCF₃, OCH₂CF₃, -OCOalkyle, -OCONRalkyle, -NRCOalkyle, NRCOOalkyle et NRCONR₂, R pouvant être, dans chacun des groupes précédents, un atome d'hydrogène ou un groupe alkyle, haloalkyle, aryle, arylalkyle, cycloalkyle ou (cycloalkyl)alkyle;
R₆ est choisi entre l'hydrogène et les groupes alkyle, OH, O-alkyle, amine, amine substitué, NHCOR (où R est tel que défini ci-dessus), CN et NO₂;
R₈ est choisi entre l'hydrogène et un groupe alkyle;
Rg est choisi entre l'hydrogène et un groupe alkyle, alcényle, aryle, arylalkyle, cycloalkyle ou cycloalkylalkyle; et
n est égal à 1,2ou3;
où:
"alkyle", "alcényle", "alcynyle" et "alcoxy" désignent de tels groupes ayant jusqu'à huit atomes de carbone;
"cycloalkyle" désigne de tels groupes ayant de trois à sept atomes de carbone;
"halo" et "halogène" désignent le chlore, le brome ou le fluor;
"aryle" désigne un groupe phényle, 1-naphtyle, 2-naphtyle ou phényle, 1-naphtyle ou 2-naphtyle monosubs- titué, ledit substituant étant un groupe alkyle de 1 à 4 atomes de carbone, alkylthio de 1 à 4 atomes de carbone, alcoxy de 1 à 4 atomes de carbone, un atome d'halogène, ou un groupe nitro, cyano, hydroxy, amine, -NH-alkyle où le groupe alkyle a de 1 à 4 atomes de carbone, -N(alkyle)₂ où le groupe alkyle a de 1 à 4 atomes de carbone, - CF₃, -OCHF₂, 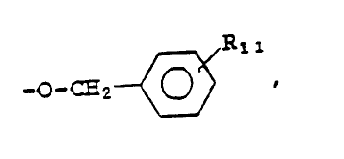 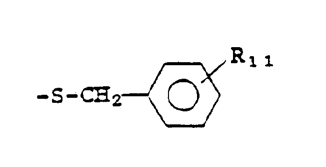 (où R₁ est l'hydrogène, un groupe alkyle de 1 à 4 atomes de carbone, alcoxy de 1 à 4 atomes de carbone, alkylthio de 1 à 4 atomes de carbone, un atome d'halogène ou un groupe hydroxy ou CF₃), -O-CH₂-cycloalkyle ou -S-CH_{z}- cycloalkyle , ou phényle, 1-naphtyle ou 2-naphtyle di-substitué, lesdits substituants étant choisis entre les groupes méthyle, méthoxy et méthylthio, les atomes d'halogène et les groupes CF₃, nitro, amine et OCHF_{z}; et
"amine substitué" désigne un groupe de formule -NZ₁Z₂ dans laquelle Z₁ est un atome d'hydrogène ou un groupe alkyle, cycloalkyle, aryle, arylalkyle ou cycloalkylalkyle, et Z₂ est un groupe alkyle, cycloalkyle, aryle, arylalkyle ou cycloalkylalkyle, ou bien Z₁ et Z₂forment, avec l'atome d'azote auquel ils sont fixés, un groupe 1-pyrrolidinyle, 1-pipéridinyle, 1-azépinyle, 4-morpholinyle, 4-thiamorpholinyle, 1-pipérazinyle, 4-alkyl-1-pipérazinyle, 4-arylalkyl-1-pipérazinyle, 4-diarylalkyl-1-pipérazinyle, ou 1-pyrrolidinyle, 1-pipéridinyle ou 1-azépinyle substitué par alkyle, alcoxy, alkylthio, halo, trifluorométhyle ou hydroxy.

2. Composé selon la revendication 1, dans lequel
R₁ est le chlore ou le fluor;
R₂ est un groupe trans-hydroxy;
R₃ et R₄ sont tous deux le groupe méthyle;
R₅ est -CN ou -N0₂;
R₆ est l'hydrogène;
R₇ est l'hydrogène;
R₈ est l'hydrogène; et
Rg est l'hydrogène.

3. Composé selon la revendication 1, ayant pour formule 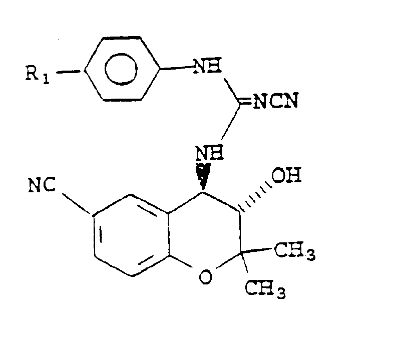 dans laquelle R est le fluor ou le chlore.

4. Composé selon la revendication 1, qui est la (trans)-N"-cyano-N-(6-cyano-3,4-dihydro-3-hydroxy-2,2-diméthyl-2H-1-benzopyran-4-yl)-N'-(4-cyanophényl)guanidine.

5. Composé selon la revendication 1, qui est la (trans)-N"-cyano-N-(6-cyano-3,4-dihydro-3-hydroxy-2,2-diméthyl-2H-1-benzopyran-4-yl)-N'-(4-méthoxyphényl) guanidine.

6. Composé selon la revendication 1, qui est la (trans)-N"-cyano-N-(6-cyano-3,4-dihydro-3-hydroxy-2,2-diméthyl-2H-1-benzopyran-4-yl)-N'-(4-nitrophényl)guanidine.

7. Composé selon la revendication 1, qui est la (trans)-N-(4-chlorophényl)-N"-cyano-N-(6-cyano-3,4-dihydro-3-hydroxy-2,2-diméthyl-2H-1-benzopyran-4-yl)guanidine.

8. Composé selon la revendication 1, qui est la (trans)-N-(2-chlorophényl)-N"-cyano-N-(6-cyano-3,4-dihydro-3-hydroxy-2,2-diméthyl-2H-1-benzopyran-4-yl) guanidine.

9. Composé selon la revendication 1, qui est la (trans)-N-(3-chlorophényl)-N"-cyano-N-(6-cyano-3,4-dihydro-3-hydroxy-2,2-diméthyl-2H-1-benzopyran-4-yl)-guanidine.

10. Composé selon la revendication 1, qui est la (trans)-N-(4-fluorophényl)-N"-cyano-N-(6-cyano-3,4-dihydro-3-hydroxy-2,2-diméthyl-2 H-1-benzopyran-4-yl)guanidine.

11. Composé selon la revendication 1, qui est la (3S-trans)-N"-cyano-N-(6-cyano-3,4-dihydro-3-hydroxy-2,2-diméthyl-2H-1-benzopyran-4-yl)-N'-(4-fluorophényl)guanidine.

12. Composé selon la revendication 1, qui est la (3S-trans)-N-(4-chlorophényl)-N"-cyano-N'-(6-cyano-3,4-dihydro-3-hydroxy-2,2-diméthyl-2H-1-benzopyran-4-yl)guanidine.

13. Composé selon la revendication 1, qui est la (3S-trans)-N-(3-chlorophényl)-N"-cyano-N'-(6-cyano-3,4-dihydro-3-hydroxy-2,2-diméthyl-2H-1-benzopyran-4-yl)guanidine.

14. Composé selon la revendication 1, qui est la trans-N"-cyano-N-(6-cyano-3,4-dihydro-3-hydroxy-2,2-diméthyl-2H-1-benzopyran-4-yl)-N'-[4-(phénylméthoxy)-phényl)]guanidine.

15. Composé selon la revendication 1, qui est la trans-N"-cyano-N-(6-cyano-3,4-dihydro-3-hydroxy-2,2-diméthyl-2H-1-benzopyran-4-yl)-N'-(4-hydroxyphényl)guanidine.

16. Composé selon la revendication 1, qui est la (3S-trans)-N-(3,4-dichlorophényl)-N"-cyano-N'-(6-cyano-3,4-dihydro-3-hydroxy-2,2-diméthyl-2H-1-benzopyran-4-yl)guanidine.

17. Composé selon la revendication 1, qui est la (3S-trans)-N"-cyano-N-(6-cyano-3,4-dihydro-3-hydroxy-2,2-diméthyl-2H-1-benzopyran-4-yl)-N'-[4-(trifluorométhyl)phényl ]guanidine.

18. Composé selon la revendication 1, qui est la (3S-trans)-N"-cyano-N-(6-cyano-3,4-dihydro-3-hydroxy-2,2-diméthyl-2H-1-benzopyran-4-yl)-N'-(4-nitrophényl)guanidine.

19. Composé selon la revendication 1, qui est la (3S-trans)-N-(3-nitrophényl-N"-cyano-N'-(6-cyano-3,4-dihydro-3-hydroxy-2,2tliméthyl-2H-1-benzopyran-4-yl)guanidine.

20. Composé selon la revendication 1, qui est la (3S-trans)-N-(3-trifluorométhylphényl)-N"-cyano-N'-(6-cyano-3,4-dihydro-3-hydroxy-2,2-diméthyl-2H-1-benzopyran-4-yl)guanidine.

21. Composé selon la revendication 1, qui est la (3S-trans)-N'-cyano-N-(6-cyano-3,4-dihydro-3-hydroxy-2,2-diméthyl-2H-1-benzopyran-4-yl)-N'-(3-méthylphényl)guanidine.

22. Composé selon la revendication 1, qui est la (3S-trans)-N"-cyano-N-(6-cyano-3,4-dihydro-3-hydroxy-2,2-diméthyl-2H-1-benzopyran-4-yl)-N'-(4-méthylphényl)guanidine.

23. Procédé de préparation d'un composé selon la revendication 1, procédé qui consiste à combiner un composé de formule 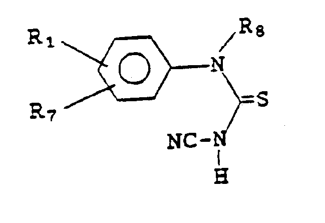 avec un composé de formule 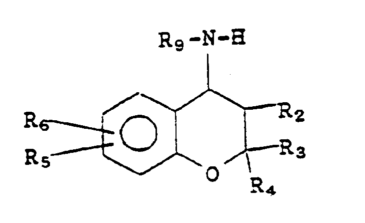 en présence d'un carbodiimide.

24. Procédé selon la revendication 23, dans lequel ledit carbodiimide a pour formule 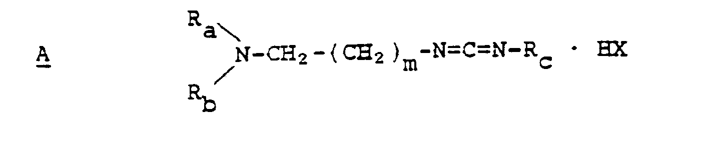 dans laquelle X est un halogène, Rₐ, R_{b} et Rₑ sont indépendamment un groupe alkyle, cycloalkyle, phényle, phé- nylalkyle ou cycloalkylalkyle, ou bien Rₐ et R_{b} forment, avec l'atome d'azote, un groupe 1-pyrrolidinyle, 1-pipéridinyle, 4-morpholinyle, 4-thiomorpholinyle, 4-alkyl-1-pipérazinyle ou 4-phénylalkyl-1-pipérazinyle.

25. Procédé selon la revendication 23, dans lequel ledit carbodiimide est le chlorhydrate de 1-(3-diméthylaminopropyl)-3-éthylcarbodiimide.

26. Procédé selon la revendication 23,24 ou 25, dans lequel le composé produit est un composé selon l'une quelconque des revendications 2 à 22.

27. Composé selon l'une quelconque des revendications 1 à 22, destiné au traitement d'états cardio-vasculaires.

28. Utilisation du composé selon l'une quelconque des revendications 1 à 22 pour la fabrication d'un médicament permettant de traiter des états cardio-vasculaires.

29. Utilisation du composé selon l'une quelconque des revendications 1 à 22 pour la fabrication d'un médicament permettant de protéger un organe d'un dommage ischémique chez une espèce mammifère sujette à une opération chirurgicale de cet organe.

30. Utilisation selon la revendication 29, dans laquelle ladite opération chirurgicale est un pontage cardio-pulmonaire.

31. Utilisation selon la revendication 29, dans laquelle ladite opération chirurgicale est une transplantation d'organe.

32. Utilisation selon la revendication 31, dans laquelle ladite opération chirurgicale est une transplantation cardiaque.

33. Utilisation selon la revendication 29, dans laquelle ledit composé est ajouté à une solution utilisée dans ladite opération pour préserver, protéger ou maintenir la fonction de l'organe.

34. Utilisation selon la revendication 33, dans laquelle une quantité cardio-protectrice dudit composé est ajoutée à une solution cardioplégique utilisée pour arrêter, perfuser, entreposer et/ou protéger un coeur impliqué dans une opération de pontage cardio-pulmonaire ou de transplantation cardiaque.

35. Utilisation selon la revendication 29, dans laquelle ledit composé est administré à une espèce mammifère en train de subir une opération chirurgicale d'organe avant et/ou pendant et/ou après ladite opération.

36. Utilisation selon les revendications 31 ou 32, dans laquelle ledit composé est administré à un donneur d'organe avant et/ou pendant et/ou après le prélèvement dudit organe dudit donneur.

37. Solution cardioplégique comprenant une quantité cardio-protectrice d'un composé selon l'une quelconque des revendications 1 à 22, et un porteur approprié pour celui-ci.

38. Procédé de protection d'un organe de mammifère d'un dommage ischémique à l'extérieur du corps, procédé qui consiste à utiliser une quantité protectrice de l'organe d'un composé selon l'une quelconque des revendications 1 à 22.

39. Procédé selon la revendication 38, dans lequel l'organe est un coeur destiné à une opération chirurgicale de transplantation cardiaque.

40. Procédé selon la revendication 38 ou 39, dans lequel ledit composé est ajouté à une solution utilisée dans ladite opération pour préserver, protéger ou maintenir la fonction de l'organe.

## Revendications (Revendications pour l'(les) Etat(s) contractant(s) suivant(s) : ES, GR)

1. Procédé de préparation d'un composé de formule 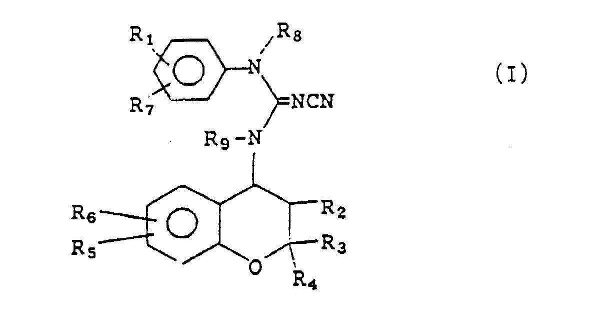
R₁ et R₇ sont chacun, indépendamment, l'hydrogène ou un substituant cyano, alcoxy, nitro, halo, hydroxy, haloalkyle ou benzyloxy, sous réserve qu'au moins l'un de R₁ et R₇ soit autre que l'hydrogène;
R₂ est l'hydrogène ou un groupe hydroxy ou 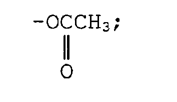
R₃ et R₄ sont chacun, indépendamment, l'hydrogène ou un groupe alkyle ou arylalkyle, ou bien R₃ et R₄ forment, avec l'atome de carbone auquel ils sont fixés, un noyau carbocyclique de 5 à 7 chaînons;
R₅ est choisi entre l'hydrogène et les substituants alkyle, haloalkyle, alcényle, alcynyle, cycloalkyle, arylalkyle, cycloalkylalkyle, -CN, -NO₂, -COR, -COOR, -CONHR, -CONR₂, -CF₃, S-alkyle, -SOalkyle, -S0₂alkyle, 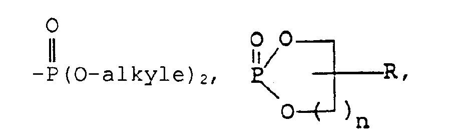 halogène, amine, amine substitué, O-alkyle, OCF₃, OCH₂CF₃, -OCOalkyle, -OCONRalkyle, -NRCOalkyle, NRCOOalkyle et NRCONR₂, R pouvant être, dans chacun des groupes précédents, un atome d'hydrogène ou un groupe alkyle, haloalkyle, aryle, arylalkyle, cycloalkyle ou (cycloalkyl) alkyle;
R₆ est choisi entre l'hydrogène et les groupes alkyle, OH, O-alkyle, amine, amine substitué, NHCOR (où R est tel que défini ci-dessus), CN et NO_{z};
R₈ est choisi entre l'hydrogène et un groupe alkyle;
Rg est choisi entre l'hydrogène et un groupe alkyle, alcényle, aryle, arylalkyle, cycloalkyle ou cycloalkylalkyle; et
n est égal à 1,2ou3;
où:
"alkyle", "alcényle", "alcynyle" et "alcoxy" désignent de tels groupes ayant jusqu'à huit atomes de carbone;
"cycloalkyle" désigne de tels groupes ayant de trois à sept atomes de carbone;
"halo" et "halogène" désignent le chlore, le brome ou le fluor;
"aryle" désigne un groupe phényle, 1-naphtyle, 2-naphtyle ou phényle, 1-naphtyle ou 2-naphtyle monosubs- titué, ledit substituant étant un groupe alkyle de 1 à 4 atomes de carbone, alkylthio de 1 à 4 atomes de carbone, alcoxy de 1 à 4 atomes de carbone, un atome d'halogène, ou un groupe nitro, cyano, hydroxy, amine, -NH-alkyle où le groupe alkyle a de 1 à 4 atomes de carbone, -N(alkyle)₂ où le groupe alkyle a de 1 à 4 atomes de carbone, - CF₃, -OCHF₂, 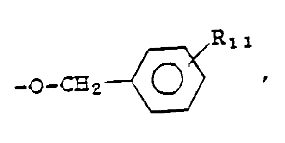 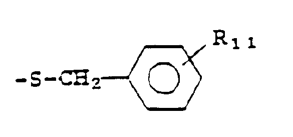 (où R₁₁ est l'hydrogène, un groupe alkyle de 1 à 4 atomes de carbone, alcoxy de 1 à 4 atomes de carbone, alkylthio de 1 à 4 atomes de carbone, un atome d'halogène ou un groupe hydroxy ou CF₃), -O-CH₂-cycloalkyle ou -S-CH₂- cycloalkyle , ou phényle, 1-naphtyle ou 2-naphtyle di-substitué, lesdits substituants étant choisis entre les groupes méthyle, méthoxy et méthylthio, les atomes d'halogène et les groupes CF₃, nitro, amine et OCHF_{z}; et
"amine substitué" désigne un groupe de formule -NZ₁Z₂ dans laquelle Z₁ est un atome d'hydrogène ou un groupe alkyle, cycloalkyle, aryle, arylalkyle ou cycloalkylalkyle, et Z₂ est un groupe alkyle, cycloalkyle, aryle, arylalkyle ou cycloalkylalkyle, ou bien Z₁ et Z₂ forment, avec l'atome d'azote auquel ils sont fixés, un groupe 1-pyrrolidinyle, 1-pipéridinyle, 1-azépinyle, 4-morpholinyle, 4-thiamorpholinyle, 1-pipérazinyle, 4-alkyl-1-pipérazinyle, 4-arylalkyl-1-pipérazinyle, 4-diarylalkyl-1-pipérazinyle, ou 1-pyrrolidinyle, 1-pipéridinyle ou 1-azépinyle substitué par alkyle, alcoxy, alkylthio, halo, trifluorométhyle ou hydroxy;
procédé qui consiste à combiner un composé de formule 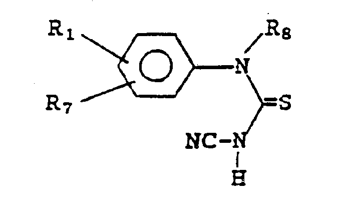 avec un composé de formule 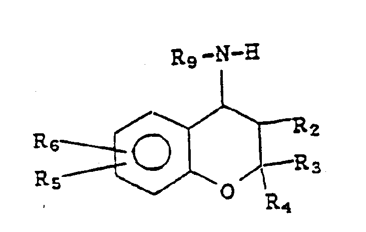 en présence d'un agent de combinaison.

2. Procédé selon la revendication 1, dans lequel
R₁ est le chlore ou le fluor;
R₂ est un groupe trans-hydroxy;
R₃ et R₄ sont tous deux le groupe méthyle;
R₅ est -CN ou -NO₂;
R₆ est l'hydrogène;
R₇ est l'hydrogène;
R₈ est l'hydrogène; et
Rg est l'hydrogène.

3. Procédé selon la revendication 1, dans lequel le produit (I) a pour formule 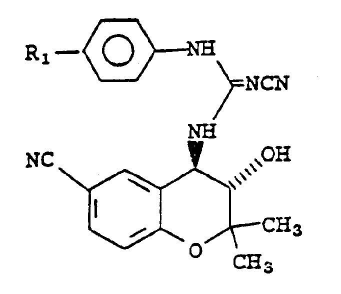 où R₁ est le fluor ou le chlore.

4. Procédé selon la revendication 1, dans lequel le produit (I) est la (trans)-N"-cyano-N-(6-cyano-3,4-dihydro-3-hydroxy-2,2-diméthyl-2H-1-benzopyran-4-yl)-N'-(4-cyanophényl)guanidine.

5. Procédé selon la revendication 1, dans lequel le produit (I) est la (trans)-N"-cyano-N-(6-cyano-3,4-dihydro-3-hydroxy-2,2tüméthyl-2H-1-benzopyran-4-yl)-N'(4-méthoxyphényl)guanidine.

6. Procédé selon la revendication 1, dans lequel le produit (I) est la (trans)-N"-cyano-N-(6-cyano-3,4-dihydro-3-hydroxy-2,2-diméthyl-2H-1-benzopyran-4-yl)-N'-(4-nitrophényl)guanidine.

7. Procédé selon la revendication 1, dans lequel le produit (I) est la (trans)-N-(4-chlorophényl)-N"-cyano-N-(6-cyano-3,4-dihydro-3-hydroxy-2,2-diméthyl-2H-1-benzopyran-4-yl)guanidine.

8. Procédé selon la revendication 1, dans lequel le produit (I) est la (trans)-N-(2-chlorophényl)-N"-cyano-N-(6-cyano-3,4-dihydro-3-hydroxy-2,2-diméthyl-2H-1-benzopyran-4-yl)guanidine.

9. Procédé selon la revendication 1, dans lequel le produit (I) est la (trans)-N-(3-chlorophényl)-N"-cyano-N-(6-cyano-3,4-dihydro-3-hydroxy-2,2-diméthyl-2H-1-benzopyran-4-yl)guanidine.

10. Procédé selon la revendication 1, dans lequel le produit (I) est la (trans)-N-(4-fluorophényl)-N"-cyano-N-(6-cyano-3,4-dihydro-3-hydroxy-2,2-diméthyl-2H-1-benzopyran-4-yl)guanidine.

11. Procédé selon la revendication 1, dans lequel le produit (I) est la (3S-trans)-N"-cyano-N-(6-cyano-3,4-dihydro-3-hydroxy-2,2-diméthyl-2H-1-benzopyran-4-yl)-N'-(4-fluorophényl)guanidine.

12. Procédé selon la revendication 1, dans lequel le produit (I) est la (3S-trans)-N-(4-chlorophényl)-N"-cyano-N'-(6-cyano-3,4-dihydro-3-hydroxy-2,2-diméthyl-2H-1-benzopyran-4-yl)guanidine.

13. Procédé selon la revendication 1, dans lequel le produit (I) est la (3S-trans)-N-(3-chlorophényl)-N"-cyano-N'-(6-cyano-3,4-dihydro-3- hydroxy-2,2-diméthyl-2H-1-benzopyran-4-yl)guanidine.

14. Procédé selon la revendication 1, dans lequel le produit (I) est la trans-N"-cyano-N-(6-cyano-3,4-dihydro-3-hydroxy-2,2-diméthyl-2H-1-benzopyran-4-yl)-N'-[4-(phénylméthoxy)phényl]guanidine.

15. Procédé selon la revendication 1, dans lequel le produit (I) est la trans-N"-cyano-N-(6-cyano-3,4-dihydro-3-hydroxy-2,2-diméthyl-2H-1-benzopyran-4-yl)-N'-(4-hydroxyphényl)guanidine.

16. Procédé selon la revendication 1, dans lequel le produit (I) est la (3S-trans)-N-(3,4-dichlorophényl)-N"-cyano-N'-(6-cyano-3,4-dihydro-3-hydroxy-2,2-diméthyl-2H-1-benzopyran-4-yl)guanidine.

17. Procédé selon la revendication 1, dans lequel le produit (I) est la (3S-trans)-N"-cyano-N-(6-cyano-3,4-dihydro-3-hydroxy-2,2-diméthyl-2H-1-benzopyran-4-yl)-N'-[4-(trifluorométhyl)phényl ]guanidine.

18. Procédé selon la revendication 1, dans lequel le produit (I) est la (3S-trans)-N"-cyano-N-(6-cyano-3,4-dihydro-3-hydroxy-2,2-diméthyl-2H-1-benzopyran-4-yl)-N'-(4-nitrophényl) guanidine.

19. Procédé selon la revendication 1, dans lequel le produit (I) est la (3S-trans)-N-(3-nitrophényl)-N"-cyano-N'-(6-cyano-3,4-dihydro-3-hydroxy-2,2-diméthyl-2H-1-benzopyran-4-yl)guanidine.

20. Procédé selon la revendication 1, dans lequel le produit (I) est la (3S-trans)-N-(3-trifluorométhylphényl)-N"-cyano-N'-(6-cyano-3,4-dihydro-3-hydroxy-2,2-diméthyl-2H-1-benzopyran-4-yl)-guanidine.

21. Procédé selon la revendication 1, dans lequel le produit (I) est la (3S-trans)-N'-cyano-N-(6-cyano-3,4-dihydro-3-hydroxy-2,2-diméthyl-2H-1- benzopyran-4-yl)-N'-(3-méthylphényl)guanidine.

22. Procédé selon la revendication 1, dans lequel le produit (I) est la (3S-trans)-N"-cyano-N-(6-cyano-3,4-dihydro-3-hydroxy-2,2-diméthyl-2H-1-benzopyran-4-yl)-N'-(4-méthylphényl)guanidine.

23. Procédé selon l'une quelconque des revendications précédentes, dans lequel l'agent de combinaison est un carbodiimide.

24. Procédé selon la revendication 23, dans lequel ledit carbodiimide a pour formule 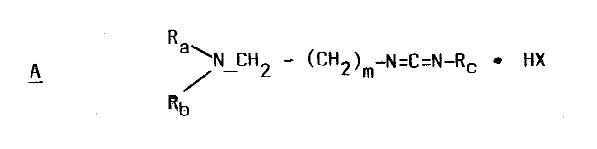 dans laquelle X est un halogène, Rₐ, R_{b} et R_{c} sont, indépendamment, un groupe alkyle, cycloalkyle, phényle, phé- nylalkyle ou cycloalkylalkyle, ou bien Rₐ et R_{b} forment, avec l'atomed'azote, un groupe 1-pyrrolidinyle, 1-pipéridinyle, 4-morpholinyle, 4-thiomorpholinyle, 4-alkyl-1-pipérazinyle ou 4-phénylalkyl-1-pipérazinyle.

25. Procédé selon la revendication 23, dans lequel ledit carbodiimide est le chlorhydrate de 1-(3-diméthylaminopropyl)-3-éthyl-carbodiimide.

26. Procédé de protection d'un organe de mammifère d'un dommage ischémique à l'extérieur du corps, procédé qui consiste à utiliser une quantité protectrice de l'organe d'un composé de formule 1 tel qu'elle est définie dans l'une quelconque des revendications 1 à 22.

27. Procédé selon la revendication 26, dans lequel l'organe est un coeur destiné à une opération chirurgicale de transplantation cardiaque.

28. Procédé selon la revendication 26 ou 27, dans lequel ledit composé est ajouté à une solution utilisée dans ladite opération pour préserver, protéger ou maintenir la fonction de l'organe.
